# EUROPEAN PATENT SPECIFICATION

(11) **EP 3 240 898 B1**
(45) Date of publication and mention of the grant of the patent: **14.08.2024**
(21) Application number: 15817414.4
(22) Date of filing: 29.12.2015
(51) Int. Cl.: C12Q 1/26, C12N 9/02, C12Q 1/6883, C12N 15/113, A61K 38/00

(54) **PRENYLCYSTEINE OXIDASE 1 INHIBITORS FOR THE PREVENTION AND/OR TREATMENT OF OXIDATIVE STRESS-RELATED DEGENERATIVE DISEASES AND PRENYLCYSTEINE OXIDASE 1 AS DIAGNOSTIC MARKER**
PRENYLCYSTEIN-OXIDASE-1-INHIBITOREN ZUR VORBEUGUNG UND/ODER BEHANDLUNG VON ERKRANKUNGEN IN VERBINDUNG MIT OXIDATIVEM STRESS UND PRENYLCYSTEIN-OXIDASE-1 ALS DIAGNOSTISCHER MARKER
INHIBITEURS DE PRÉNYLCYSTÉINE OXYDASE 1 POUR LA PRÉVENTION ET/OU LE TRAITEMENT DE MALADIES DÉGÉNÉRATIVES LIÉES AU STRESS OXYDATIF ET PRÉNYLCYSTÉINE OXYDASE 1 UTILISÉE COMME MARQUEUR DE DIAGNOSTIC

(30) Priority: 29.12.2014 EP 14200476
(43) Date of publication of application: 08.11.2017
(73) Proprietor: Centro Cardiologico Monzino S.p.A- IRCCS, 20138 Milano (IT)
(72) Inventor: BANFI, Cristina, 20138 Milano (MI) (IT); BAETTA, Roberta, 20138 Milano (MI) (IT); BRIOSCHI, Maura, 20138 Milano (MI) (IT); BARBIERI, Silvia, Stella, 20138 Milano (MI) (IT)
(74) Representative: Capasso, Olga
(86) International application number: PCT/EP2015/081354
(87) International publication number: WO 2016/107874

(56) References cited:
- QAMAR W ET AL: "Farnesol ameliorates massive inflammation, oxidative stress and lung injury induced by intratracheal instillation of cigarette smoke extract in rats: An initial step in lung chemoprevention", CHEMICO-BIOLOGICAL INTERACTIONS, ELSEVIER SCIENCE IRLAND, IR, vol. 176, no. 2-3, 25 November 2008 (2008-11-25), pages 79 - 87, XP025628001, ISSN: 0009-2797, [retrieved on 20080828], DOI: 10.1016/J.CBI.2008.08.011
- CRISTINA BANFI ET AL: "Proteomic analysis of human low-density lipoprotein reveals the presence of prenylcysteine lyase, a hydrogen peroxide-generating enzyme", PROTEOMICS, vol. 9, no. 5, 1 March 2009 (2009-03-01), DE, pages 1344 - 1352, XP055261140, ISSN: 1615-9853, DOI: 10.1002/pmic.200800566
- J. A. DIGITS ET AL: "Stereospecificity and Kinetic Mechanism of Human Prenylcysteine Lyase, an Unusual Thioether Oxidase", JOURNAL OF BIOLOGICAL CHEMISTRY, vol. 277, no. 43, 25 October 2002 (2002-10-25), US, pages 41086 - 41093, XP055261144, ISSN: 0021-9258, DOI: 10.1074/jbc.M208069200
- DATABASE EMBL [online] 18 April 2011 (2011-04-18), "WO 2005116204-A/226088: Double strand polynucleotides generating RNA interference.", XP002755925, retrieved from EBI accession no. EM_PAT:FW819562 Database accession no. FW819562

## Description

### BACKGROUND OF THE INVENTION

Genetic, clinical and pharmacological studies implicate elevated plasma levels of low density lipoproteins (LDL) in the pathogenesis of atherosclerotic vascular disease, the leading cause of death in industrialized societies. However, at any level of plasma cholesterol, there is a wide variation in the incidence of coronary artery disease. It is evident that, besides cholesterol, many factors might affect the lipoprotein-arterial wall interaction, thus exacerbating the atherogenic process. Among these factors, lipoprotein modifications, mainly oxidation, are crucial for the uptake of LDL by cells in the first stages of atherogenesis. Overproduction of free radicals can cause oxidative damage to biomolecules, eventually leading to many chronic diseases (oxidative stress-related degenerative diseases) such as atherothrombosis, atherosclerosis, diabetes, cardiovascular diseases, chronic inflammation, stroke and septic shock, cancer, aging and other degenerative diseases in humans (Reuter, Gupta et al. 2010).

However, the nature of these modifications and the determinants, either lipoprotein intrinsic or extrinsic factors, remain unclear.

By proteomics, the authors discovered that prenylcysteine oxidase 1 (PCYOX1; alternative name prenylcysteine lyase, PCL, NCBI PROTEIN ACCESSION GI:166795301, NP_057381.3), an enzyme involved in the degradation of prenylated proteins, is associated with atherogenic lipoproteins and, in the presence of its substrate, can generate hydrogen peroxide, H₂O₂ (Banfi, Brioschi et al. 2009).

Prenylation is a class of lipid modification involving covalent addition of either farnesyl (15-carbon) or geranylgeranyl (20-carbon) isoprenoids to conserved cysteine residues at or near the C-terminus of proteins. Known prenylated proteins include fungal mating factors, nuclear lamins, Ras and Ras-related GTP-binding proteins (G proteins), the subunits of trimeric G proteins, protein kinases, and at least one viral protein. Prenylation promotes membrane interactions of most of these proteins, which is not surprising given the hydrophobicity of the lipids involved. In addition, however, prenylation appears to play a major role in several protein-protein interactions involving these species (Zhang and Casey 1996).

Lu JY and Hofmann SL (Lu and Hofmann 2006) details metabolic pathways for the lysosomal degradation of S-fatty acylated and prenylated proteins. Central to these pathways are two lysosomal enzymes, palmitoyl-protein thioesterase (PPT1) and PCYOX1. PPT1 is a soluble lipase that cleaves fatty acids from cysteine residues in proteins during lysosomal protein degradation. Notably, deficiency in the enzyme causes a neurodegenerative lysosomal storage disorder, infantile neuronal ceroid lipofuscinosis. PCYOX1 is a membrane-associated flavin-containing lysosomal monooxygenase that metabolizes prenylcysteine to prenyl aldehyde through a completely novel mechanism. The eventual metabolic fates of other lipidated proteins (such as glycosylphosphatidylinositol-anchored and N-myristoylated proteins) are poorly understood, suggesting directions for future research.

In *in vitro* experiments, PCYOX1 cleaves the thioether bond of prenylcysteines to yield free cysteine and the aldehyde of the isoprenoid lipid (Tschantz, Digits et al. 2001). However, the importance of this enzyme has not yet been fully defined at the biochemical or physiologic level. Beigneux A, *et al* (Beigneux, Withycombe et al. 2002) show that PCYOX1 is expressed at high levels in mouse liver, kidney, heart, and brain. To test whether PCYOX1 deficiency would cause prenylcysteines to accumulate in tissues and result in pathologic consequences, they produced PCYOX1-deficient cell lines and PCYOX -deficient mice (PCYOX1^{-/-}). PCYOX1 activity levels were markedly reduced in PCYOX1^{-/-} cells and tissues. PCYOX1^{-/-} fibroblasts were more sensitive than wild-type fibroblasts to growth inhibition when prenylcysteines were added to the cell culture medium. To determine if the reduced PCYOX1 enzyme activity levels led to an accumulation of prenylcysteines within cells, mass spectrometry was used to measure farnesylcysteine and geranylgeranylcysteine levels in the tissues of PCYOX1^{-/-} mice and wild-type controls. These studies revealed a striking accumulation of both farnesylcysteine and geranylgeranylcysteine in the brain and liver of PCYOX1^{-/-} mice. This accumulation did not appear to be accompanied by significant pathologic consequences. PCYOX1^{-/-} mice were healthy and fertile, and surveys of more than 30 tissues did not uncover any abnormalities. They conclude that prenylcysteine lyase does play a physiologic role in cleaving prenylcysteines in mammals, but the absence of this activity does not lead to major pathologic consequences.

A clinical trial of a protein farnesyltransferase (FTase) inhibitor (the small-molecule, lonafarnib) for the treatment of Hutchinson-Gilford progeria syndrome (HGPS) was recently completed (Young, Yang et al. 2013). FTase is a cytosolic enzyme that adds a 15-carbon farnesyl lipid to a diverse group of protein substrates, including the Ras proteins, a family of small guanosine triphosphatases (GTPase) involved in normal signal transduction and cancer development.

Farnesyl transferase inhibitors (FTIs) have so far proved to have limited value as single agents in clinical trials. Wojtkowiak JW, et al. (Wojtkowiak, Gibbs et al. 2009) focus on the use of a novel group of FTIs that are most effective *in vitro* when used in combination with the "statin" class of anti-hypercholesterolemic agents, which also block protein prenylation. They recently showed that these novel FTIs in combination with lovastatin reduce Ras prenylation and induce an apoptotic response in malignant peripheral nerve sheath cells. The combination of statins with these new FTIs may produce profound synergistic cytostatic and cytotoxic effects against a variety of tumors and other proliferative disorders. Since statins are well tolerated in the clinic, they suggest that this combination approach should be tested in *in vivo* models.

WO02072811 refers to reagents that regulate human prenylcysteine lyase-like protein and reagents which bind to human prenylcysteine lyase-like protein gene products that can play a role in preventing, ameliorating, or correcting dysfunctions or diseases including cancer.

US2014274964 refers to combination therapies for the treatment of progeroid diseases and conditions, cellular aging, bone diseases, and cardiovascular diseases. The provided combination therapies target the aberrant farnesylation of the mutant lamin A protein progerin, and inhibit farnesyl biosynthesis, lamin A prenylation, and increase progerin clearance.

Among available models of atherosclerosis, the apolipoprotein E-deficient (apoE^{-/-}) mouse is particularly popular because of its propensity to spontaneously develop atherosclerotic lesions on a standard chow diet (Meir and Leitersdorf 2004). The apoE^{-/-} mice available today through The Jackson Laboratories are descendants of the original apoE^{-/-} mouse created by the Maeda group (t002052 B6.129P2-Apoetm1Unc).

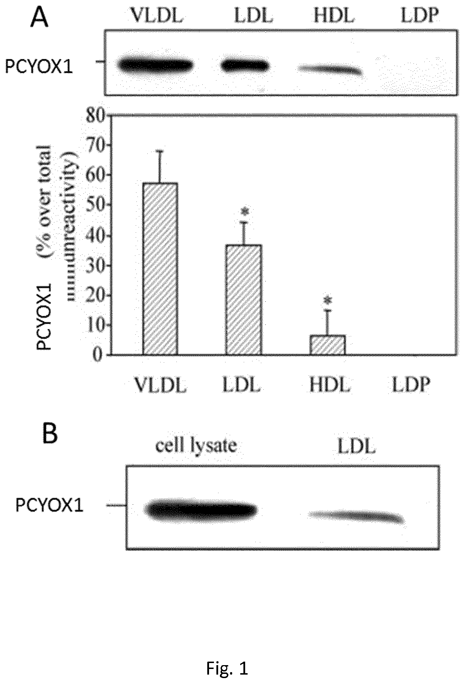

Proteomic analysis of human low-density lipoprotein reveals the presence of PCYOX1, a hydrogen peroxide-generating enzyme (Banfi, Brioschi et al. 2009). In details, the authors discovered that PCYOX1 is associated with atherogenic lipoproteins. To analyze the distribution of PCYOX1 by immunoblotting in the lipoprotein classes, VLDL, LDL, and HDL were isolated by ultracentrifugation from the plasma of normolipidemic healthy subjects. PCYOX1 content declined from VLDL to LDL to HDL, and was absent from lipoprotein-depleted plasma (Fig. 1A). PCYOX1 is generated as part of the synthesis of nascent lipoprotein and is not merely captured from the blood; HepG2 cells, a popular model used for studies on lipoprotein metabolism, express PCYOX1 protein and release it in association with lipoprotein (Fig. 1B).

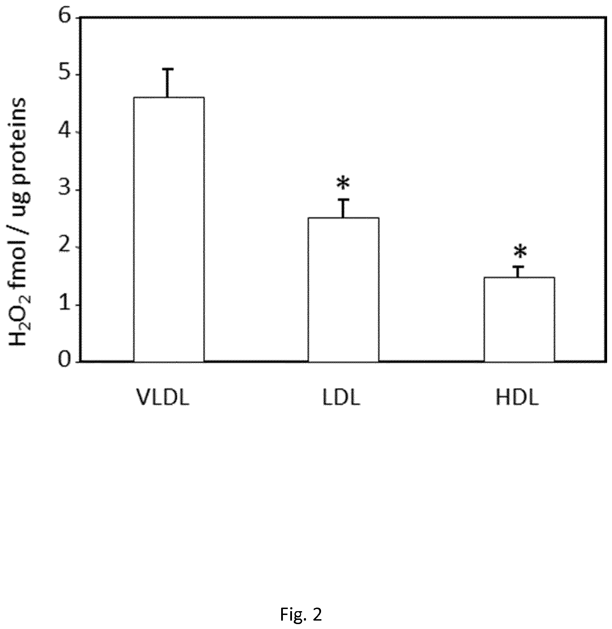

To determine whether lipoprotein PCYOX1 produce H₂O₂ in a cell-free system, a peroxidase-linked assay employing a nonfluorogenic substrate that is converted to a fluorogenic compound by horseradish peroxidase (HRP) when H₂O₂ is present, was used (Zhou, Diwu et al. 1997). The incubation of VLDL, the major carrier of PCYOX1, with the enzyme substrate farnseylcysteine (FC) in the presence of HRP and Amplex Red resulted in progressive generation of H₂O₂ (Fig. 2). This phenomenon was more marked with VLDL than with LDL or HDL, reflecting the finding that the PCYOX1 protein content is highest in VLDL. Overall, these findings stress the importance of understanding both PCYOX1 biology and mechanism(s) of action, which are still unknown, and might point out an important biological role for this enzyme in the development of atherosclerosis.

As stated above PCYOX1, previously named Pcly, has been identified as lysosomal enzyme that catalyzes the degradation of prenylcysteines and their methyl esters (Zhang L. 1997; Digits, Pyun. 2002). The basic features of the chemical mechanism through which Pcly catalyzes its reaction have been elucidated (Tschantz et al. 2001). The main features are as follows: (i) the products of the reaction are free cysteine and the C-1 aldehyde of the isoprenoid moiety (farnesal or geranylgeranial); (ii) the enzyme utilizes molecular oxygen as a co-substrate; (iii) Pcly uses a noncovalently bound FAD cofactor in an NAD(P)H-independent manner; and (iv) a stoichiometric amount of hydrogen peroxide is produced during the course of the reaction.

Regarding the measurement of the activity, the amount and the alteration of PCYOX, methods available aims at determine the enzymatic activity of purified bovine Pcly by assay aimed at evaluating the conversion of Pcly substrate FC to cysteine and the generation of hydrogen peroxide. Conventional methods will be discloses below.

In the case of evaluating the conversion of Pcly substrate FC to cysteine, the conversion of [³⁵S]FC (~50,000 cpm/time point) to [³⁵S]cysteine by purified Pcly is generally monitored by thin-layer chromatography (Zhang L. 1997), and by a fluorimetric assay to assess cysteine production. This latter involves reacting the cysteine formed during the reaction with the fluorogenic compound 7-diethylamino-3-(4'-maleimidylphenyl)-4-methylcoumarin to yield a fluorescent product. Pcly (300 nmol/L) is mixed with saturating FC (100 µmol/L) in 50 mM sodium phosphate, pH 7.4, at 25 °C. At time intervals ranging to 30 min, 50-µL samples are removed and mixed with 50 µL of 0.2 mmol/L 7-diethylamino-3-(4'-maleimidylphenyl)-4-methylcoumarin in the assay buffer. A control reaction without FC is processed in parallel. The intensity of fluorescence is determined (λex = 384 nm; λem = 470 nm) in a 100-µL cuvette, and cysteine formed is calculated from a cysteine standard curve.

Similarly, Pcly activity in cell or tissue extracts may be assessed by thin-layer chromatography. Briefly, 10 µg of protein was incubated for 30 min at 37 °C in 50 mmol/L Tris (pH 7.7) containing 10 µmol/L [³⁵S]farnesylcysteine (~40,000 dpm/reaction) in a final volume of 20 µL The reaction is stopped by adding 10 µL of the thin-layer chromatography solvent (n-propanol:NH₄OH:H₂O (6:3:1, v/v)). Samples are processed by silica-gel thin-layer chromatography with a method that separates the reaction product, [³⁵S]cysteine, from the substrate, [³⁵S]famesylcysteine. To assess the amount of [³⁵S]cysteine in each reaction, the plates are exposed to x-ray film (Beigneux, Withycombe et al. 2002).

Hydrogen peroxide produced in the reaction by purified bovine Pcly is generally measured as reported (Tschantz, Digits et al. 2001) using the Amplex Red kit (Molecular Probes). Purified Pcly (10 ng/µL) is incubated at 23 °C in the buffer provided, which also contains horseradish peroxidase and Amplex Red. After the base line is established, the Pcly substrate FC is added to a final concentration of 5 µmol/L. The action of horseradish peroxidase on Amplex Red in the presence of hydrogen peroxide converts the Amplex Red into a fluorogenic product. Fluorescence analysis of the reaction mixture is then conducted (λex = 563 nm, λem = 587 nm).

The disclosed conventional assays suffer from the fact that are expensive, time consuming and limited by need of radioactive compounds, low reproducibility, not high throughput, and performed on purified Pcly. Furthermore, they measure Pcly activity and not protein amount.

### SUMMARY OF THE INVENTION

It is therefore an object of the invention a molecule able to inhibit or block PCYOX1 expression and/or function for use in the treatment and/or prevention of oxidative stress related degenerative diseases selected from the group consisting of: atherothrombosis, atherosclerosis, diabetes, cardiovascular diseases, chronic inflammation, stroke and septic shock, cancer, aging, preferably of atherothrombosis. Preferably, the molecule is able to inhibit the PCYOX1 functions e.g. the reaction leading to H₂O₂. The only known inhibitors of the PCYOX1 function, e.g. molecules inhibiting the PCYOX1 reaction leading to H₂O₂, are represented by farnesal and by the prenyl alcohol farnesol which can be considered a dead end inhibitor (Digits, Pyun et al. 2002).

Therefore preferred molecules according to the invention are farnesal and farnesol. In a preferred embodiment of the invention, PCYOX1 is a protein and it preferably has essentially the amino acid sequence disclosed as NCBI PROTEIN ACCESSION GI:166795301, NP_057381.3 or of SEQ ID NO:3.

In another preferred embodiment, PCYOX1 is a gene and it preferably has essentially the nucleotide sequence disclosed as NCBI Accession no. gi|568815596:70258099-70281185 or of SEQ ID NO:1. In another preferred embodiment, PCYOX1 is a mRNA and it preferably has essentially the nucleotide sequence disclosed as NCBI Accession no.: NM_016297.3 or of SEQ ID NO:2.

Preferably, the molecule according to the invention is selected from the group consisting of:
a) polynucleotide able to specifically inhibit the expression of the gene PCYOX1 selected from a siRNA, a shRNA, a microRNA or an antisense oligonucleotide;
b) a vector comprising or expressing said polynucleotide of a);
c) farnesal or farnesol.

Preferably said polynucleotide able to inhibit the expression of the gene PCYOX1 is an RNA inhibitor, more preferably it is a siRNA, a shRNA, a microRNA or an antisense oligonucleotide.

In a more preferred embodiment, the shRNA comprises the SEQ ID NO: 6 or 9 or 12 or a functional derivative thereof wherein the functional derivative inhibits the expression of the PCYOX1 gene. In another preferred embodiment, the siRNA comprises at least one of the sequences selected from the group consisting of the SEQ ID NO: 7, SEQ ID NO: 8, SEQ ID NO: 10, SEQ ID NO: 11, SEQ ID NO: 13 or SEQ ID NO: 14, or a functional derivative thereof wherein the functional derivative inhibits the expression of the PCYOX1 gene.

In a more preferred embodiment of the invention, the molecule is a shRNA consisting of the SEQ ID NO: 6, SEQ ID NO: 9 or SEQ ID NO: 12, or a functional derivative thereof or a siRNA consisting of the SEQ ID NO: 7, SEQ ID NO: 8, SEQ ID NO: 10, SEQ ID NO:11, SEQ ID NO: 13 or SEQ ID NO: 14, or a functional derivative thereof wherein the functional derivative inhibits the expression of the PCYOX1 gene.

In a preferred embodiment, the above vector is an expression vector selected from the group consisting of: plasmids, viral particles and phages.

Another object of the invention is a composition comprising the molecule as above defined combined with at least one anti-atherothrombotic agent, e.g. agents able to reduce low density lipoprotein, lipoprotein(a), triglyceride-rich lipoprotein and/or at least one agent able to modulate high density lipoprotein levels and function (see e.g. Do, Nicholls et al. 2014) and/or at least one anticoagulants and antiplatelet drugs (see e.g. Bhatt, Hulot et al. 2014) for use in the treatment and/or prevention of oxidative stress-related degenerative diseases selected from the group consisting of: atherothrombosis, atherosclerosis, diabetes, cardiovascular diseases, chronic inflammation, stroke and septic shock, cancer, aging, preferably of atherothrombosis.

The oxidative stress-related degenerative disease is selected from the group consisting of: atherothrombosis, atherosclerosis, diabetes, cardiovascular diseases, chronic inflammation, stroke and septic shock, cancer, aging and other degenerative diseases (Reuter, Gupta et al. 2010).

A further object of the invention is a pharmaceutical composition comprising the above molecule or the composition as above defined and at least one pharmaceutically acceptable carrier, for use in the treatment and/or prevention of oxidative stress-related degenerative diseases selected from the group consisting of: atherothrombosis, atherosclerosis, diabetes, cardiovascular diseases, chronic inflammation, stroke and septic shock, cancer, aging, preferably of atherothrombosis.

Another object of the invention is a genetically engineered host cell transduced, transformed or transfected with and able to express in suitable conditions the shRNA or the siRNA or a functional derivative thereof as defined above for use in the treatment and/or prevention of oxidative stress-related degenerative diseases, preferably of atherothrombosis.

Preferably, said host cell is selected from the group consisting of: bacterial cells, fungal cells, insect cells, animal cells, plant cells, preferably being an animal cell, more preferably a human cell.

It is herein disclosed not as part of the invention a method, preferably in vitro, for measuring total activity of PCYOX1 protein and/or to measure the alteration of the activity of PYCOX1 protein comprising the steps of:
- isolating lipoprotein fraction from a biological sample;
- incubating the isolated lipoprotein fraction with famseylcysteine;
- measuring the amount of H₂O₂ generated
wherein the incubation time is preferably of 20 minutes to six hours, preferably of two hours.

The PCYOX1 protein is preferably plasmatic.

Preferably, the FC is used in a concentration from 30 to 250 µmol/L, preferably of 125µmol/L.

The density of the lipoproteins fraction is preferably of 1.006-1.25 g/mL, more preferably of 1.21 g/mL.

It is herein disclosed not as part of the invention a method, preferably in vitro, for detecting and/or to measuring the amount and/or measuring the alteration of the amount of PYCOX1 protein or of fragments thereof comprising the steps of:
a) digestion of the PCYOX1 protein in a biological sample to obtain PCYOX1 fragments;
b) quantification of PCYOX1 fragments by mass spectrometry.

Preferably, PCYOX1 fragments are:
i) peptides 7-25 amino acids long and/or
ii) peptides that are detectable by LC-MS and/or
iii) peptides that do not contain missed cleavage sites and/or
iv) peptides that do not contain residues susceptible to artefactual modifications,
preferably said fragments comprise at least one of the peptide comprising the SEQ ID NO: 4 or SEQ ID NO:5.

The above methods may comprise a step a') before step b) wherein the PCYOX1 fragments are chromatographically separated.

It is herein disclosed not as part of the invention anin vitro method for assessing the risk and/or for diagnosing and/or for prognosing and/or for monitoring the progression and/or for monitoring the efficacy of a therapeutic treatment and/or for the screening of a therapeutic treatment of an oxidative stress-related degenerative disease, preferably of atherothrombosis, in a subject comprising the steps of:
a) detecting or measuring the amount or the activity of the protein PCYOX1 or of fragments thereof or the polynucleotide coding for said protein or fragments thereof in an isolated biological sample obtained from the subject and
b) comparing with respect to a proper control.

In the above method, preferably step a) comprises measuring the amount of the protein PCYOX1 or of fragments thereof or of the polynucleotide coding for said protein or of fragments thereof in said isolated biological sample obtained from the subject and step b) comprises comparing the measured amount of step a) with a proper control amount.

Preferably, the in vitro method for assessing the risk and/or diagnosing and/or prognosing of an oxidative stress-related degenerative disease, preferably of atherothrombosis, as above defined, comprises the steps of:
a) measuring the amount or the activity of the protein PCYOX1 or of fragments thereof or of the polynucleotide coding for said protein or fragments thereof in said isolated biological sample obtained from the subject and
b) comparing the measured amount or activity of step a) with a proper control amount or activity,
wherein an amount or activity of said protein PCYOX1 or of fragments thereof or of said polynucleotide or of fragments thereof in the isolated biological sample obtained from the subject higher than the control amount or activity indicates that the subject is either at increased risk for developing or is affected by the oxidative stress-related degenerative disease.

Preferably, the in vitro method for monitoring the progression and/or for monitoring the efficacy of a therapeutic treatment of an oxidative stress-related degenerative disease, preferably of atherothrombosis, as above defined, comprises the steps of:
a) measuring the alteration of the amount or the alteration of the activity of the protein PCYOX1 or of fragments thereof or of the polynucleotide coding for said protein or fragments thereof in said isolated biological sample obtained from the subject and
b) comparing the measured alteration of step a) with a proper control alteration.

In a preferred aspect, the step a) of the above defined methods is carried out by the method for measuring total activity of PCYOX1 protein and/or to measure the alteration of the activity of PYCOX1 protein or by the method for detecting and/or to measuring the amount and/or measuring the alteration of the amount of PYCOX1 protein or of fragments thereof as above defined.

Preferably, the biological sample is a fluid, a cell or a tissue sample, more preferably said sample is plasma or serum.

It is herein disclosed not as part of the invention a kit for carrying out the above methods, comprising
- means to measure the amount or the activity of the protein PCYOX1 or of fragments thereof and/or means to measure the amount of the polynucleotide coding for said protein or of fragments thereof and optionally,
- control means.

It is herein disclosed not as part of the invention a method for treating a subject affected by an oxidative stress-related degenerative disease, preferably by atherothrombosis, comprising administering to said subject an effective amount of at least one molecule as above defined or the pharmaceutical composition as above defined.

It is also herein disclosed not as part of the invention an in vitro method for identifying SNPs (single nucleotide polymorphisms) and/or other gene variants (e.g. aminoacids-changing SNPs) in PCYOX1 gene responsible for vascular events in oxidative stress-related degenerative diseases, preferably atherothrombosis.

By the term "molecule able to inhibit or block" it is meant a molecule that effects a change in the expression and/or function of the target. The change is relative to the normal or baseline level of expression and/or function in the absence of the molecule, but otherwise under similar conditions, and it represent a decrease in the normal/baseline expression and/or function.

The terms "function" and "activity" are herein interchangeable.

The inhibition or blocking of the expression and/or function of the target may be assessed by any means known to the skilled in the art. The assessment of the expression level or of the presence of the target is preferably performed using classical molecular biology techniques such as (real time Polymerase Chain Reaction) qPCR, microarrays, bead arrays, RNAse protection analysis or Northern blot analysis or cloning and sequencing.

The assessment of target function (or activity) is e.g. performed by assaying the generation of an oxidant or lipoprotein oxidation, e.g. by incubating human lipoproteins (as e.g. VLDL) with FC and analysing the increase of electrophoretic mobility on agarose gel due to oxidation. In order to assess target function, hydrogen peroxide (H₂O₂) produced in the PCYOX1 reaction can be measured as previously described (Tschantz, Digits et al. 2001), or with methods herein disclosed.

In the context of the present invention, the target is the gene, the mRNA, the cDNA, or the encoded protein thereof.

The polynucleotides as above described, as e.g. the siRNAs, may further comprise dTdT or UU 3'-overhangs, and/or nucleotide and/or polynucleotide backbone modifications as described elsewhere herein. The siRNA according to the invention may comprise nucleotides 1-19 of SEQ ID NO: 7, 8, 10, 11, 13 or 14.

As used herein, the term " genetically engineered host cell " relates to host cells which have been transduced, transformed or transfected with the polynucleotide or with the vector described previously. As representative examples of appropriate host cells, one can cite bacterial cells, such as E. coli, Streptomyces, Salmonella typhimurium, fungal cells such as yeast, insect cells such as Sf9, animal cells such as CHO or COS, plant cells, etc. The selection of an appropriate host is deemed to be within the scope of those skilled in the art from the teachings herein. Preferably, said host cell is an animal cell, and most preferably a human cell.

Further assessment methods include in vitro suppression assay, whole transcriptome analysis, mass spectrometry analysis to identify proteins interacting with the target.

In the present invention, a method of reducing PCYOX1 protein amount is also provided, wherein it is used a PCYOX1 inhibitor, e.g. a short hairpin RNA (shRNA).

The shRNA used in the present invention is preferably a pool of 3 different shRNA plasmids: sc-76093-SHA:
Hairpin sequence:
   GATCCGGAAAGATGTGAAGATAGATTCAAGAGATCTATCTTCACATCTTTCCTTTTT (SEQ ID NO: 6)
Corresponding siRNA sequences (sc-76093A):
   - Sense: GGAAAGAUGUGAAGAUAGAtt (SEQ ID NO: 7)
   - Antisense: UCUAUCUUCACAUCUUUCCtt (SEQ ID NO: 8)
sc-76093-SHB:
   Hairpin sequence:
      GATCCCCACTCCGTTGAATCGAAATTCAAGAGATTTCGATTCAACGGAGTGGTTTTT (SEQ ID NO: 9)
   Corresponding siRNA sequences (sc-76093B):
      - Sense: CCACUCCGUUGAAUCGAAAtt (SEQ ID NO: 10)
      - Antisense: UUUCGAUUCAACGGAGUGGtt (SEQ ID NO: 11)
sc-76093-SHC:
   Hairpin sequence:
      GATCCGAAGCCCAATCTGTATCAATTCAAGAGATTGATACAGATTGGGCTTCTTTTT (SEQ ID NO: 12)
   Corresponding siRNA sequences (sc-76093C):
      - Sense: GAAGCCCAAUCUGUAUCAAtt (SEQ ID NO: 13)
      - Antisense: UUGAUACAGAUUGGGCUUCtt (SEQ ID NO: 14)

All sequences are provided in 5' ---> 3' orientation.

The polynucleotide according to the invention includes also chemically and structurally modified polynucleotide, preferably siRNAs, to solve their intrinsic problems related to in vivo instability, off-target effects, and immunogenicity.

The vector according to the invention also includes novel vector-driven bifunctional short hairpin RNA (bi-shRNA) (Ku SH., et al. 2015; Mohr SE,et al. 2014).

The vector of the invention also include an RNAi-inducing vector whose presence within a cell results in production of an siRNA or shRNA or miRNA, preferably siRNA.

One or more siRNAs or shRNAs (in any combination) targeting PCYOX1 may be used.

Such polynucleotides can be single or double stranded. Preferably, one strand of a double-stranded polynucleotide comprises at least a partial sequence complementary to a target mRNA. The nucleotides of the inhibitory nucleic acid can be chemically modified, natural or artificial. The sequence homology between the polynucleotide (e.g. a RNAi inducing agent) and the targeted PCYOX1 target mRNA may be 100% or less, but is ideally greater than about 50% and typically 90% or greater and even more preferably at least 98% and 99%. It will be understood that the percentage of sequence homology between RNAi inducing agent and the target mRNA should be sufficient to result in sequence specific association between the RNAi inducing agent, e.g. siRNA, and the target mRNA, preferably under cytoplasmic conditions.

Such siRNAs comprise two RNA strands having a region of complementarity of approximately 20 or so nucleotides in length and optionally further comprises one or two single-stranded overhangs or loops. In mammalian cells, dsRNA longer than 30 base pairs can cause non-specific gene suppression by an interferon a response. However, cells transfected with 21 nucleotide synthetic double-stranded siRNA bearing two nucleotides protruding at both 3'-ends have been found to escape an interferon response and effectively exert sequence-specific gene silencing function. The silencing effect of the synthetic siRNA, however, is transient. The double stranded siRNA molecule down regulates expression of the PCYOX1 protein via RNAi, wherein each strand of said siRNA molecule is independently about 18 to about 28 nucleotides in length and one strand of the siRNA molecule comprises a nucleotide sequence having sufficient complementarity to the RNA of the target protein for the siRNA molecule to direct cleavage of the target RNA via RNA interference. In shRNA, the single RNA strand may form a hairpin structure with a stem and loop and, optionally, one or more unpaired portions at the 5' and/or 3' portion of the RNA.

The delivery of an effective amount of the polynucleotide according to the invention, may be carried out with any know method which is suitable for the delivery. For example, some delivery agents for the siRNA, microRNA, etc. are selected from the following non-limiting group of cationic polymers, modified cationic polymers, peptide molecular transporters, lipids, liposomes and/or non-cationic polymers. Viral vector delivery systems may also be used. For example, an alternative delivery route includes the direct delivery of the polynucleotide (including siRNA, shRNA and miRNA) and even anti-sense RNA (asRNA) in gene constructs followed by the transformation of cells with the resulting recombinant DNA molecules. This results in the transcription of the gene constructs encoding the polynucleotide, such as siRNA, shRNA and miRNA, or even asRNA and provides for the transient and stable expression of the polynucleotide in cells and organisms. For example, such an alternative delivery route may involve the use of a lentiviral vector comprising a nucleotide sequence encoding a siRNA (or shRNA) which targets the PCYOX1. Such a lentiviral vector may be comprised within a viral particle. Adeno-associated viruses (AAV) may also be used and the use of these as delivery vehicles is expanded on later. siRNA delivery formulations include e.g. siRNA conjugates, polymerized siRNA, and nucleic acid-based nanoparticles to improve in vivo delivery.

RNA interference (RNAi) is the process by which expression of a target gene is effectively silenced or knocked down by the selective inactivation of its corresponding mRNA by double-stranded RNA (dsRNA). RNAi is activated by dsRNA species delivered to the cytoplasm of cells. The silencing mechanisms can either lead to the degradation of a target mRNA, as induced by small interfering RNAs (siRNAs) or short hairpin RNAs (shRNAs), or the suppression of translation of specific mRNAs, as induced by microRNA (miRNA). Two key approaches to RNAi that have gained substantial interest for use in gene silencing are the double-stranded small interfering RNAs (siRNAs) and the vector-based short hairpin RNAs (shRNAs).

Both these methods can achieve robust and specific knockdown, but they have striking mechanistic differences with broad practical implications. shRNA can effectively target knockdown with low copy numbers and longer lasting effects than siRNA. The recently introduced bifunctional design has similar benefits to standard shRNA but with greatly enhanced potency.

The use of shRNA is suggested by the advantages of shRNA over siRNA including the ability to use viral vectors for delivery to overcome the difficulty of transfecting certain cell types, the option to control shRNA expression using inducible promoters, and the ability to co-express them with a reporter gene. Additionally, they may cause fewer off-target effects.

Furthermore, novel vector-driven bifunctional short hairpin RNA (bi-shRNA) technology that harnesses both cleavage-dependent and cleavage-independent RISC loading pathways to enhance knockdown potency may be used. Indeed, consequent advantages provided by the bi-shRNA include a lower effective systemic dose than comparator siRNA/shRNA to minimize the potential for off-target side effects, due to its ability to induce both a rapid (inhibition of protein translation) and delayed (mRNA cleavage and degradation) targeting effect depending on protein and mRNA kinetics, and a longer duration of effectiveness for clinical applications.

Measurement of a protein amount using a workflow based exclusively on the use of mass spectrometry, like Multiple Reaction Monitoring (MRM) represents an alternative to conventional assays usually used in the clinical setting. Conventional protein assays typically rely on antibody recognition systems coupled to a variety of detection modalities (e.g., enzymatic amplification, electrochemiluminescence, and fluorescence) but they suffer from the fact that are expensive, time consuming and limited by the lack of specificity and the inability to detect protein isoforms. On the other hand, MRM relies on the monitoring, by mass spectrometry, of the levels of selected quantotypic peptides specific for the protein of interest obtained after protein digestion. Indeed, treatment of proteins with a selective enzyme ( i.e. trypsin) generates a set of peptides representing the peptide mass fingerprinting of the protein.

Results from the MRM assay can be used to correlate accurate and precise quantitative levels of the PCYOX1 protein with the specific oxidative stress-related disease of the patient from whom the sample (e.g. plasma, blood, tissue, and cells) was collected. This not only provides diagnostic information about the disease, but also permits a physician or other medical professional to determine appropriate therapy for the patient. For example, such an assay can be designed to diagnose the stage or degree of a disease and determine which therapeutic agent, or course of therapy, to which a patient is most likely to respond with a positive outcome. In clinical research, the MRM potentialities promise to reduce time and inaccuracies in the sample processing steps for the validation of panels of relevant and novel biomarkers for which specific antibodies or reagents are not available. Moreover, using heavy isotope-labeled peptides/ proteins added to biological samples is possible to quantify endogenous targets and also evaluate with precision the isoforms and post-translational modifications; this is usually difficult with the currently available biochemical techniques based on immunoassay.

In the MRM analysis, the prepared sample is ionized, by electrospray ionization, and the resultant ions are analyzed by mass spectrometry to detect and quantify two or more surrogate peptides present in the sample. Mass spectrometric analysis of the prepared sample may be carried out in any instrument capable of monitoring multiple transitions in MRM mode, such as a triple quadrupole mass spectrometer.

Triple quadrupole mass spectrometers utilize two resolving quadrupoles, also referred to as quadrupole mass filters, each of which is operable to selectively transmit only ions having a mass-to-charge ratio (m/z) of interest. An RF-only quadrupole filled with a collision gas (a collision cell) is interposed in the ion path between the two resolving quadrupoles. Precursor ions selected in the first resolving quadrupole undergo energetic collisions in the collision cell to yield fragment ions. The fragment ions pass into the second resolving quadrupole, and the selectively transmitted product ions strike a detector, which generates a signal representative of the quantity of transmitted ions. A specified pair of precursor/product ions to which the first and second resolving quadrupoles are tuned is referred to as a transition. The number of transitions that may be concurrently monitored in a given time period will depend on various operational factors, based on the characteristics of the commercially available instruments, that are typically capable of monitoring a few hundred transitions.

Due to the multiplicity of charge states and fragmentation that exist for each peptide fragment, monitoring all possible precursor-to-product ion transitions for the group of surrogate peptides is generally impractical. Instead, the mass spectrometer is operated to monitor a subset of transitions selected to optimize sensitivity and selectivity for each surrogate peptide. In a typical assay, a set of 2-4 transitions are monitored for each targeted peptide.

The selection of optimized transitions for particular surrogate peptides may be performed in an automated or semi-automated fashion using MS-dedicated software, which identifies and refines an optimized set of transitions based on the expected transition intensity (determined using fragmentation rules and/or previously acquired data) and potential interferences (arising for example, from co-eluting peptide fragments derived from the sample matrix or other surrogate peptide fragments).

An MRM assay can measure relative or absolute levels of specific unmodified and modified peptides from a protein.

Relative quantitation is achieved by comparing the chromatographic peak area for a tryptic peptide to the peak area of the same tryptic peptide, in other samples derived from different and separate biological sources, normalizing to total amount of proteins analyzed in each sample.

Comparison of the chromatographic peak area for a peptide from the protein of interest to the peak areas from other peptides derived from different proteins within the same sample can be performed to normalize changing levels of one protein to levels of other proteins that do not change their expression in the analysed set of samples (i.e, other plasma proteins).

Absolute quantitation of a given peptide is achieved by comparing the peak area for a given peptide in an individual biological sample to the peak area of an internal standard analyzed simultaneously. The internal standard can be a labeled version (i.e., a labeled synthetic peptide) consisting of the exact amino acid sequence of the peptide that is being interrogated. The labeled standard is spiked into a sample in known amounts, and the chromatographic peak area can be determined for both the internal peptide standard and the native peptide in the biological sample separately. Comparison of both peak areas allows the calculation of the absolute molarity or absolute amount of the native peptide present in the original protein preparation from the biological sample, that reflects the concentration or amount of the protein of interest.

Absolute quantitation can be performed across many peptides, and thus proteins, at the same time in a single sample and/or across many samples.

Control means can be used to compare the amount or the increase of amount of the compound as above defined to a proper control. The proper control may be obtained for example, with reference to known standard, either from a normal subject or from normal population.

The means to measure the amount of at least one compound as above defined are preferably at least one antibody, functional analogous or derivatives thereof. Said antibody, functional analogous or derivatives thereof are specific for said compound.

The kit described not as part of the invention comprises:
- a solid phase adhered antibody specific for said compound;
- detection means of the ligand specific-biomarker complex. Other means may be e.g. specific primers and probes for RT PCR. Means able to measure the activity of PCYOX1 may be e.g. FC and means to measure the amount of H₂O₂, labeled peptides (as e.g. the peptides as defined above) to measure the amount of PCYOX1 by MRM.

The kits described herein can further comprise customary auxiliaries, such as buffers, carriers, markers, etc. and/or instructions for use.

In the case of a method or a kit for assessing the risk and/or diagnosing and/or prognosing of an oxidative stress-related degenerative disease, the proper control may be a sample taken from a healthy patient or from a patient affected by another disorder or pathology not characterized by oxidative stress, and the proper control amount or activity may be the amount or activity of the same protein or polynucleotide measured in a sample taken from a healthy patient or from a patient affected by another disorder or pathology not characterized by oxidative stress.

In the case of a method or a kit for monitoring the progression of the oxidative stress-related degenerative disease, the progress of the disease is monitored and the proper control may be a sample taken from the same subject at various times or from another patient, and the proper control amount or activity may by the amount or activity of the same protein or polynucleotide measured in a sample taken from the same subject at various times or from another patient.

In the case of a method or a kit for monitoring the efficacy of a therapeutic treatment, the proper control may by a sample taken from the same subject before initiation of the therapy or taken at various times during the course of the therapy and the proper control amount or activity may be the amount or activity of the same protein or polynucleotide measured in a sample taken from the same subject before initiation of the therapy or taken at various times during the course of the therapy.

In the case of a method or a kit for the screening of a therapeutic treatment, the proper control may be a sample taken from subjects without treatment and from subjects treated with a substance that is to be assayed or from subjects treated with a reference treatment and the proper control amount or activity may be the average of the amounts or activity of the same protein or polynucleotide measured in samples taken from subjects without treatment and from subjects treated with a substance that is to be assayed or from subjects treated with a reference treatment. In this case, if the amount or activity of said protein PCYOX1 or of said polynucleotide in the isolated biological sample obtained from the subject is lower or equal than the control amount or activity, it may indicate that the tested substance is effective for the treatment of the oxidative stress-related degenerative disease.

In the present description, the expression "measuring the amount" can be intended as measuring the amount (or the activity) or concentration or level of the respective protein and/or mRNA thereof and/or DNA thereof, preferably semi-quantitative or quantitative. Measurement of a protein can be performed directly or indirectly. Direct measurement refers to the amount or concentration measure of the biomarker, based on a signal obtained directly from the protein, and which is directly correlated with the number of protein molecules present in the sample. This signal - which can also be referred to as intensity signal - can be obtained, for example, by measuring an intensity value of a chemical or physical property of the biomarker. Indirect measurements include the measurement obtained from a secondary component (e.g., a different component from the gene expression product) and a biological measurement system (e.g. the measurement of cellular responses, ligands, "tags" or enzymatic reaction products).

The term "amount", as used in the description refers but is not limited to the absolute or relative amount of proteins and/or mRNA thereof and/or DNA thereof, and any other value or parameter associated with the same or which may result from these. Such values or parameters comprise intensity values of the signal obtained from either physical or chemical properties of the protein, obtained by direct measurement, for example, intensity values in an immunoassay, mass spectroscopy or a nuclear magnetic resonance. Additionally, these values or parameters include those obtained by indirect measurement, for example, any of the measurement systems described herein. Methods of measuring mRNA and DNA in samples are known in the art. To measure nucleic acid levels, the cells in a test sample can be lysed, and the levels of mRNA in the lysates or in RNA purified or semi-purified from lysates can be measured by any variety of methods familiar to those in the art. Such methods include hybridization assays using detectably labeled DNA or RNA probes ( i.e., Northern blotting) or quantitative or semi-quantitative RT-PCR methodologies using appropriate oligonucleotide primers. Alternatively, quantitative or semi-quantitative in situ hybridization assays can be carried out using, for example, tissue sections, or unlysed cell suspensions, and detectably labeled (e.g., fluorescent, or enzyme-labeled) DNA or RNA probes. Additional methods for quantifying mRNA include RNA protection assay (RPA), cDNA and oligonucleotide microarrays, representation difference analysis (RDA), differential display, EST sequence analysis, and serial analysis of gene expression (SAGE).

If by comparing the measured amount or activity of the protein PCYOX1 or of the polynucleotide coding for said protein with the amount or activity obtained from a control sample, the amount or the activity of said compound in the sample isolated from the subject corresponds to a higher value, the subject may present the disease or go towards an aggravation of said disease.

If by comparing the measured amount or activity of the protein PCYOX1 or of the polynucleotide coding for said protein with the amount or the activity obtained from a control sample, the amount or the activity of said compound in the sample isolated from the subject corresponds to a similar or lower value, the subject may be not affected by the disease or go toward an amelioration of the disease, respectively.

Alternatively, the expression "detection" or "measuring the amount" is intended as measuring the alteration of the molecule. Said alteration can reflect an increase or a decrease in the amount or activity of the molecules as above defined. An increase of the protein or of the activity of PCYOX1 or of the polynucleotide coding for said protein can be correlated to an aggravation of the disease. A decrease of the protein or of the activity of PCYOX1 or of the polynucleotide coding for said protein can be correlated to an amelioration of the disease or to recovery of the subject.

The expression "protein PCYOX1" or "PCYOX1" is intended to include also the corresponding protein encoded from a PCYOX1 orthologous or homologous genes, functional mutants, functional derivatives, functional fragments or analogues, isoforms thereof.

The expression "gene PCYOX1" or "PCYOX1" is intended to include also the corresponding orthologous or homologous genes, functional mutants, functional derivatives, functional fragments or analogues, isoforms thereof.

In the present invention "functional mutants" of the protein are mutants that may be generated by mutating one or more amino acids in their sequences and that maintain their activity e.g. of degradation of prenylcysteines with generation of H₂O₂. Indeed, the protein of the invention, if required, can be modified in vitro and/or in vivo, for example by glycosylation, myristoylation, amidation, carboxylation or phosphorylation, and may be obtained, for example, by synthetic or recombinant techniques known in the art.

In the present invention "functional" is intended for example as "maintaining their activity" e.g. of degradation of prenylcysteines with generation of H₂O₂.

The term "analogue" as used herein referring to a protein means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide.

The term "derivative" as used herein in relation to a protein means a chemically modified peptide or an analogue thereof, wherein at least one substituent is not present in the unmodified peptide or an analogue thereof, i.e. a peptide which has been covalently modified. Typical modifications are amides, carbohydrates, alkyl groups, acyl groups, esters and the like. As used herein, the term "derivatives" also refers to longer or shorter polypeptides having e.g. a percentage of identity of at least 41 % , preferably at least 41.5%, 50 %, 54.9% , 60 %, 61.2%, 64.1%, 65 %, 70 % or 75%, more preferably of at least 85%, as an example of at least 90%, and even more preferably of at least 95% with PCYOX1, or with an amino acid sequence of the correspondent region encoded from a PCYOXlorthologous or homologous gene.

As used herein "fragments" refers to polypeptides having preferably a length of at least 10 amino acids, more preferably at least 15, at least 17 amino acids or at least 20 amino acids, even more preferably at least 25 amino acids or at least 37 or 40 amino acids, and more preferably of at least 50, or 100, or 150 or 200 or 250 or 300 or 350 or 400 or 450 or 500 amino acids.

The RNAi inhibitors as above defined are preferably capable of hybridizing to all or part of specific target sequence of PCYOX1. Therefore, RNAi inhibitors may be fully or partly complementary to all of or part of the target sequence

The RNAi inhibitors may hybridize to the specified target sequence under conditions of medium to high stringency.

An RNAi inhibitors may be defined with reference to a specific sequence identity to the reverse complement of the sequence to which it is intended to target. The antisense sequences will typically have at least about 75%, preferably at least about 80%, at least about 85%, at least about 90%, at least about 95% or at least about 99% sequence identity with the reverse complements of their target sequences. The antisense nucleic acid may be Gapmers.

A "derivative" may be a nucleic acid molecule, as a DNA molecule, coding the polynucleotide as above defined, or a nucleic acid molecule comprising the polynucleotide as above defined, or a polynucleotide of complementary sequence. In the context of the present invention the term "derivatives" also refers to longer or shorter polynucleotides and/or polynucleotides having e.g. a percentage of identity of at least 41 % , 50 %, 60 %, 65 %, 70 % or 75%, more preferably of at least 85%, as an example of at least 90%, and even more preferably of at least 95% with SEQ ID NOs: 6-14 or with their complementary sequence or with their DNA or RNA corresponding sequence. The term "derivatives" and the term "polynucleotide" also include modified synthetic oligonucleotides. The modified synthetic oligonucleotide are preferably LNA (Locked Nucleic Acid), phosphoro-thiolated oligos or methylated oligos, morpholinos, 2'-O-methyl, 2'-O-methoxyethyl oligonucleotides and cholesterol-conjugated 2'-O-methyl modified oligonucleotides (antagomirs).

The term "derivative" may also include nucleotide analogues, i.e. a naturally occurring ribonucleotide or deoxyribonucleotide substituted by a non-naturally occurring nucleotide. The modified nucleotide analog may be located for example at the 5'-end and/or the 3 '-end of the nucleic acid molecule. Nucleotide analogs may be selected from sugar- or backbone-modified ribonucleotides or nucleobase-modified ribonucleotides, i.e. ribonucleotides, containing a non-naturally occurring nucleobase instead of a naturally occurring nucleobase.

The term "derivatives" also includes nucleic acids that may be generated by mutating one or nucleotide in their sequences, equivalents or precursor sequences. The term "derivatives" also includes at least one functional fragment of the polynucleotide.

In the context of the present invention "functional" is intended for example as "maintaining their activity".

As used herein "fragments" refers to polynucleotides having preferably a length of at least 1000 nucleotides, 1100 nucleotide, 1200 nucleotides, 1300 nucleotides, 1400 nucleotides, 1500 nucleotides. Where the fragments are referred to the siRNA as above defined, the length is preferably of 10, 11, 12, 13, 14, 15, 16, 17, 18,19, 20, 21 nucleotides.

The term "polynucleotide" also refers to modified polynucleotides.

According to the present invention, an "effective amount" of a composition is one that is sufficient to achieve a desired biological effect, in this case an amelioration or the treatment of an oxidative stress-related degenerative disease.

It is understood that the effective dosage will be dependent upon the age, sex, health, and weight of the recipient, kind of concurrent treatment, if any, frequency of treatment, and the nature of the effect desired. The provided ranges of effective doses of the inhibitor or molecule of the invention (e.g. from 1 mg/kg to 100 mg/kg, in particular systemically administered) are not intended to limit the invention and represent preferred dose ranges. However, the preferred dosage can be tailored to the individual subject, as is understood and determinable by one of skill in the art, without undue experimentation.

The administration of oligonucleotides of the present invention may be carried out by known methods, wherein a nucleic acid is introduced into a desired target cell in vitro or in vivo.

An aspect of the present invention comprises a nucleic acid construct comprised within a delivery vehicle. A delivery vehicle is an entity whereby a nucleotide sequence can be transported from at least one media to another. Delivery vehicles may be generally used for expression of the sequences encoded within the nucleic acid construct and/or for the intracellular delivery of the construct. It is within the scope of the present invention that the delivery vehicle may be a vehicle selected from the group of RNA based vehicles, DNA based vehicles/vectors, lipid based vehicles, virally based vehicles and cell based vehicles. Examples of such delivery vehicles include: biodegradable polymer microspheres, lipid based formulations such as liposome carriers, coating the construct onto colloidal gold particles, lipopolysaccharides, polypeptides, polysaccharides, pegylation of viral vehicles.

In one embodiment of the present invention may comprise a virus as a delivery vehicle, where the virus may be selected from: adenoviruses, retroviruses, lentiviruses, adeno-associated viruses, herpesviruses, vaccinia viruses, foamy viruses, cytomegaloviruses, Semliki forest virus, poxviruses, RNA virus vector and DNA virus vector. Such viral vectors are well known in the art.

Commonly used gene transfer techniques include calcium phosphate, DEAE-dextran, transfection, electroporation and microinjection and viral methods. Another technique for the introduction of DNA into cells is the use of cationic liposomes. Commercially available cationic lipid formulations are e.g. Tfx 50 (Promega) or Lipofectamin 2000 (Life Technologies).

The above pharmaceutical compositions are preferably for systemic, oral, locally, preferably rectally, or topical administration.

The compositions of the present invention may be in form of a solution, e.g. an injectable solution, a cream, ointment, tablet, suspension or the like. The composition may be administered in any suitable way, e.g. by injection, particularly by intraocular injection, by oral, topical, nasal, rectal application etc. The carrier may be any suitable pharmaceutical carrier. Preferably, a carrier is used, which is capable of increasing the efficacy of the RNA molecules to enter the target-cells. Suitable examples of such carriers are liposomes, particularly cationic liposomes.

The recombinant expression vector of the invention can be any suitable recombinant expression vector, and can be used to transform or transfect any suitable host. Suitable vectors include those designed for propagation and expansion or for expression or both, such as plasmids and viruses. The recombinant expression vectors of the invention can be prepared using standard recombinant DNA techniques. Constructs of expression vectors, which are circular or linear, can be prepared to contain a replication system functional in a prokaryotic or eukaryotic host cell. Replication systems can be derived, e.g., from CoIEl, 2 µ plasmid, λ, SV40, bovine papilloma virus, and the like. Desirably, the recombinant expression vector comprises regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA- based. The recombinant expression vector can include one or more marker genes, which allow for selection of transformed or transfected hosts. Marker genes include biocide resistance, e.g., resistance to antibiotics, heavy metals, etc., complementation in an auxotrophic host to provide prototrophy, and the like. Suitable marker genes for the inventive expression vectors include, for instance, neomycin/G418 resistance genes, hygromycin resistance genes, histidinol resistance genes, tetracycline resistance genes, and ampicillin resistance genes. The recombinant expression vector can comprise a native or normative promoter operably linked to the nucleotide sequence encoding the PCYOX1 inhibitor (including functional portions and functional variants thereof), or to the nucleotide sequence which is complementary to or which hybridizes to the nucleotide sequence encoding the RNA. The selection of promoters, e.g., strong, weak, inducible, tissue- specific and developmental-specific, is within the ordinary skill of the artisan. Similarly, the combining of a nucleotide sequence with a promoter is also within the skill of the artisan. The promoter can be a non-viral promoter or a viral promoter, e.g., a cytomegalovirus (CMV) promoter, an SV40 promoter, an RSV promoter and a promoter found in the long-terminal repeat of the murine stem cell virus.

The inventive recombinant expression vectors can be designed for either transient expression, for stable expression, or for both. Also, the recombinant expression vectors can be made for constitutive expression or for inducible expression.

In the above compositions further materials as well as processing techniques and the like may be set out in Part 5 of Remington's Pharmaceutical Sciences, 20th Edition, 2000, Marck Publishing Company, Easton, Pennsylvania.

The compounds of this invention can also be administered in sustained release forms or from sustained release drug delivery systems. A description of representative sustained release materials can also be found in the incorporated materials in Remington's Pharmaceutical Sciences. Furthermore, pharmaceutical formulations can be prepared using a process, which is generally known in the pharmaceutical art.

In the present invention, when the molecule of the invention is administered with another therapeutic agent, it may be administered simultaneously or sequentially.

The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like.

A "sample" in the context of the present teachings refers to any biological sample that is isolated from a subject. A sample can include, without limitation an aliquot of body fluid, whole blood, serum, plasma, solid tissue samples such as tissue biopsies, or tissue cultures or cells derived therefrom and the progeny thereof, synovial fluid, lymphatic fluid, ascites fluid, and interstitial or extracellular fluid. The term "sample" also encompasses the fluid in spaces between cells, including gingival crevicular fluid, bone marrow, cerebrospinal fluid (CSF), saliva, mucous, sputum, semen, sweat, urine, or any other bodily fluids. "Blood sample" can refer to whole blood or any fraction thereof, including serum and plasma. Samples can be obtained from a subject by means including but not limited to venipuncture, excretion, ejaculation, massage, biopsy, needle aspirate, lavage, scraping, surgical incision, or intervention or other means known in the art. The definition also includes samples that have been manipulated in any way after their procurement, such as by treatment with reagents; washed; or enrichment for certain cell populations, such as cancer cells or samples in which lipoproteins, preferably VLDL, are isolated (e.g. by ultracentrifugation) and then analyzed. The definition also includes sample that have been enriched for particular types of molecules, e.g., nucleic acids, polypeptides, etc.

In other implementation of the methods of the invention, isolated lipoprotein classes (VLDL, LDL, HDL) from human and non human specie may be used.

In the methods of the invention, the subject (or patient) may be an animal, preferably a mammalian, or a human. The subject is preferably a human. In the case of non human specie, the proteotypic peptides for measuring PCYOX1 by means of MRM might be different and may be selected by the skilled in the art.

### SEQUENCES

The nucleotide sequence of PCYOX1 is:
>gi|568815596:70258099-70281185 Homo sapiens chromosome 2, GRCh38 Primary Assembly

GCGCAGCCGCGCAGCGGTGGGAGGACTGCGGGGCTCTTGAGGCCAGCTGCAGAGCTTGTGGAGGCCATGG
GGCGCGTCGTCGCGGAGCTCGTCTCCTCGCTGCTGGGGTTGTGGCTGTTGCTGTGCAGCTGCGGATGCCC
CGAGGGCGCCGAGCTGCGTGCTCCGCCAGATAAAATCGGTAGGCGAGAAGGGGGCGGCGCGGGAAGGTGC
TGGAGCGCGCCCCGCGCCGGGCGGCCGCTGCGCAGTGCGCCCAGATCCCACAGCCGCGACGCAGTCCAGC
GGTGCAGGCCGAGCCAGCTGCGCAGGTCGCGCTTTCCCCCATTCACACTTCAGGGCGGCTTTCAGCTCTG
GTCGGAATAGGACTGTGCATTCCCAGGCGTGGAGAGGTGCCCACTTGAGGAATGGGCGTGGACGGGGACA
GGGGCGGGCGGGCAGGCAGGCGCCTAGCGTACCTGTAGCCCCTGTCTCTTAGGTGTGGGGCTCCGGGGAG
GCCTAGGTTTCTCTATCTTCCCGTTGAACACTGACCGTGAAGACTCCAGTGTTCTGGCTTGACCTGGGGC
GCCCGGCCAGATTAACAGGCTCACGCAGGCACAGCACAGGGAGGAGTGCAAACAAGTTGACCCTGCCTGC
TTCCCTTGGGAGGCTGAACCGCCTTCCCCCAGCTCCGCTTTGCTGAGGTCGGCCTGCTCTCAGAGGCCCT
TGGCTTGCACAGTTTCTCCGCCTAGGACTAACACAGCTGCAGAAAGAATCTGTTCTCTTTCCACTGTCAA
GAGCTCCAGTGTCAGGAGCCTTGGCTCTTTGCTTCCTGTCTCCCTCCCGCCCCCCTTTAAAGTTGTGTTC
TTCGGTATCATTAACCTATCTACATTGAATCTAGACACAACAAAGCTAGGCATAAATGGCCGTCACTTTA
TTAAAAATGCGTGCTGGAAAGGTCCACCTCTAATGTGCCCGTGAGTTGGGCATCTGGAAGGGTGTGCAGG
CCCCTTGGTTCCTCCATAAAGACCTGTGAGAATCTTTGGGCATCTGGAAAAAAAATCGAGTTAATGTCAA
TATGTTAATTAATCTACTGCTTTTGTCTGGGAACAGTGTCTGTCCAGATTCAGGGAGTTAGACCTTCGCT
GCAGGTGTCACTCTTCCCCCACCAAATTGTTGGTAATAGCTTTGAGTAACAACCTCTGCTATTGATGGTC
TTTCAAAAACAAAACAACTCACAGAGCTGGTAAAAGGGGAAAATATAATCTTCTGTTTTTTTCCCTCATA
GCGATTATTGGAGCCGGAATTGGTGGCACTTCAGCAGCCTATTACCTGCGGCAGAAATTTGGGAAAGATG
TGAAGATAGACCTGTTTGAAAGAGAAGAGGTCGGGGGCCGCCTGGCTACCATGATGGTGCAGGGGCAAGA
ATACGAGGCAGGAGGTTCTGTCATCCATCCTTTAAATCTGCACATGAAACGTTTTGTCAAAGACCTGGGT
ATGTAATTTTGGTCTTGGAGCTCACCAGATTACTGTGTGACATCCCCGGATATTTTTATCAGATGGAGAA
CTGAAATTTCTGCCTTGTTAATTGCCTTACAGAGACGTTGGTAAAACTGTTTTCCTCACTTTCTTGTTAG
TTGGTACTGCCTGAGTTAGGAGCAGTTCTGCTGTAATGGGACACTAGTTGTCTGTATCTTCAGTTATCTG
GAATTACAGCCATGGGGTGGGGGTGGGAATGGCTGCCATTGTCTTTTTTTTTCTTTTGAGGAGTTTCGCT

CTTGTCGCTCAGGCTGGAGTGCAATGGTGCGATCTTGGCTCACTGCAACCTCTGCCTCCCGGGTTCAAGC
GATTCTCCTGCCTTAGCCTCCTGAGTAGCTGGGATTACAGGTGCCTGCCACCACGCCCAGCTAATTTTTG
TATTTTCAGTAGTGACGGGGTTTCACCATGTTGGCCAGCTGGTCTTGAACTCCTGACCTCAGGTGATCCG
CCTGCCTTGGCCTTCCAAAGTGCTGGGATTACAGGGGTGAGCCACCGTGCCCGGCCTCTGTTGTCTGTTC
TTAAAATTCTGGGTTTGTGTTAAAGAATCAGAGAAGGTTTGGAGATAGTCATCTCATTGTGATCATTTTG
CAGCTGAGTAAATGAGACCCAGAGACTTGAATTGTCTGGCTCAAAGTCACTCACATATGGCCATGGCCAG
GGTCAGTGGCTCATGCCTGTAATCCCAGCACTTTGGGAGGCCGAGGCAGAGGATCACTTGAGCCCAGGAG
TTAGAAACCAGTCTGGGCAACATAGTGAGATCCTGTCTTTACTAAAAATAAAAAAGTTAGCCTGGTGTGA
TGGCACAAACCTGTAGTCCCAGCTTCTTGGGAGGCAGAGGCAGGAGGATCCCTTGAGCCTGGGAGATAGA
AGCTGCAGTGAGCCTTGCATGCCACTGCACTCCAGCCTGGGCAACAAAGTGAGACCCTATCAAAACAAAC
CACCACCAACAAAACAAACTCCGTCACACAGCTGTGGATGCAGACACGTGCCTAGAACTTGGGATGTGCA
ACTGCTGCCCTGACAGCAGAGGAGCTGGATAGGTCATGGGGAAAGTCTGACAGGCCTCTGGAGGCCCTTT
CGGCAGGGAGTATTTTGTAACTTTGGCCTCCTTCCAGTTATCTGAAATATCCAGTTTTGCAAAATAGGAT
TCTCACAGGAATGGAAGTGCCTCAGCTGCCTTCCACATCCTCTGTAGATGCTGCCTCTGCTTCCGTTTCC
TCCACGAGGGCGAAGGTATACCCAGAACAAATTATGCCACGAAGATGCATGTCTGACATCTTTGTTTTTA
AATTGAATTCAGTTGGTACTCTGGCTTTTTTCCTTTGACTATCCAGTTTTTTTAGACTTTTAGTCTAAAA
ATAGCCAGGTAAAAATATATCTACTTTTGATATATTAATATTTACATGATCATCTGACCCATCGCCTAGC
AAATCTGCTTTTGGACAGTGTAGTGAAAGTATAGTGTTTCACACTGTTCCAAAGATAGAGCTCCTTTATG
TGTCTCCTGAGAACTGAGGGAAGCCCCAAATTCACTCAGATTACGTTCTTTCATTTGATCAGCATAGACA
CCACTGACTTAGCTGTGCTTTATGTTTTTGCAGGTCTCTCTGCTGTTCAGGCCTCTGGTGGCCTACTGGG
GATATATAATGGAGAGACTCTGGTATTTGAGGAGAGCAACTGGTTCATAATTAACGTGATTAAATTAGTT
TGGCGCTATGGATTTCAATCCCTCCGTATGCACATGTGGGTAGAGGACGTGTTAGACAAGTTCATGAGGT
AATTTTTTTTCCTTCCATTTAACCTAAGATCTTTTAATTTAGGTTAAATTAAACTTGATGAACTTTAGGA
ATTATGATTTCTTGTAAGCATTTTCTACCTCACAGCAAGGCCTGTTTATAACATTGTTAGGCAAAATGTT
TTGTAGAAACTATGCAGACTATTTGCTTGGATATTGAAAGGTTGGTTTATTTTTGCTTCTGAAGTGTATT
TTGCAAATGCTTCTTTAGTGTTGCCAGATACTGTTAGATGCCTTGAATTCTTATGACAACTTACTCTGGT
AGGTACTGTTTTTAGCTTCTTTAAACAGATGCAGAAGGTGGCGGTGTAGAGAGGTTTTAAAAGGCAAGTT
CACCCACATAGTAAGTGGTATAGCTGGAGTTGCCACCCAGGCAGTCTGGCCTTTGAGTCTTCACTCTTAA
TCTCTATGCTGTGCTGGTGTTGTGTATATGAATTGAGATAATGTGTACAAGAGCTTGTAGAAAGGATCAG
AAAACTTAAATGGCCTTTAATAGTGACATGTTTTCAGTGAACCAGCAGCCTGAAGTAGAGTAAAAGTTCC
ATATTCCAAGAATTTACATTCTAGCACTGTTTTGGGTAGTGAGAATTCAGGTGCCTCTTTTTCGTCCTTA
TGCTTTTCTATATTTAAACAGTTTTTTTAATGCGTGTGAATATGTATCACTTTGGTAATCAGGAAGACTA
ACATAAAATAGCCAGTCTAATTTTGAAAGGTACAATCTGTATTTTTAAAGCTGTTGGCATGTTCTAGTTT
TTTAGTTTTTTTGTTTTTTTGTTTTTGAGATGGAGTTTCGCTTGTTGCCTAGGCTGGAGTGCAATGGTTC
GATATTGGCTCACTGCAACCTCTGCCTCTTGGGTTCAAGCGATACTCCTGCCTCAGGCTCCCGAGTAGCT
GGGATTACAGATGCCTGCCACCACGCCCAGCTAATTTTTGTATTTTTAGTAGAGATGGGGTTTCACCCTC
TTGGCCAGGCTGGTCTCGAACTCCTGACCTCAGGTGATCCACCTGCCTCGGCCTCCCAAAGTGCTGGGAT
TACAGGTGTGAGCTACCGAGCTTGGCCGGCATGTGCTAATTTTTTTTTTTTTTTTTTTTGAGATGGAGTT
TTGCTCTGTCGCCCGGGCTGGAGTGCAGTGGCACCATCTCGGCTCACTGCAACCTCTGCCTCCCTGGTTC
AAGCGATTCTCCTGCCTCAGACTCCCGAATAGCTGGGATTACAGGTGCCTGCCACTGTGCCTGGCTAATT
TTTTGTATTTTTAGTAGAGACGGGGTTTCACTGTGTTAGCCAGGATGGTCTTGATCTCCTGACCTCGTGA
TCCGCCTGCCTCAGCCTCCCAAAGTGTTGGGATTGCAGGCATGAGCCACTGTGCCCGGCTAGCACATGCT
AATTTTTATGCTGCTTTACAGTTTTAGGTACTGGAGGCCAGGATCGCATCATTACACTCCAGCCTGGGTG
ACAGAACAAGATTCCATCTTGAGGGGAAAAACAAAACAAAACAAAACATGTCATATGACCAGGTATTGAA
CAGGACAAATATGAGTTAAGCAGGGCTGGGCACAGTGGCTTATGCCTCTAATCCCAGAATTTTGGGAGGC
TGAGGCTGGTGGATCACCTGAGGTCAGGAGTTTGAGACCAGCCTGACCAACATGGTAAAACCCTGTCTCT
ACAAAAAATATAAAAATTTAGCCGGCGTGGTGGTGCATGCCTGTAATCCCAGCTACTTGTGAGCCTGAGG
CAGGAGAATCACTTGAACCTGGGAGGCAGAGGTTGCAGTGAGCCACGATCGTGCCATTACACTCTAGCCT
GGGGGACAGAGCCAGACTGTCTAAAAAAAAAAAAAAGAAAGCTAAGCAGGATATGCTCTGGTTTCTATAA
CTCATGGTAGTTTGGGGTTTCTTTCTTTCTCAACATCATTCTTAGGTCAGTTCCTTAAATGCCTTTCCCG
TGGGTACCTTAAGTCCATCTACAGTGCATAATGTTTTTAAGTAGCCATTCATTCATTCACCCAATATTGA
ATACCTGTTAGGTGCTAGGCATTGTTCTATGTTGAGAGTATAGGACCTACAACATGTCCTTGTCCTCAAA
GAATATATATTCTAGTGGAGAGACAGGCAATACACAACCAAGGAAATATATAGAATTTGCGTAGAGACGG
CCAGTCACTTTATAGAAGGTGGGTGGCATGGTGCTGCATACTCAGCAGGCAGATCAAGTTCTGATGGCAT
CAGCCCTGCTTGTGTGTATTGTTTTTCTTTTCTTTTCTTTTTTTTTTTTGAGATAGAGTCTCGTTTTGTC
GCCCAGGCTGGAGTGCAGTGGCGGGATCTCGGCTTACTGCAACATCTGCCTCCCAGGTTCAAGTGATTCT
CCTGCCTCAGCCTCCTGAGTAGCTGGGACTACAGGCACATGCCACCATGCCTGGTTAATTTTTTGTATTT
TTAGTAGAGATGTGGTTTCACCGTGTTAGCCAGGCTGGTCTCAAACTCCTGACCTTGTGATCTGCCTGTC
TCGGCTTCCCAAAGTGTTGGGATTATAGGCGTGAGCCACTGTGCCCGGCCCTCTTTTTTTTTTTTTTTTT
TTTGATACAGGTCTCACTCTGTCACCCAGGCTGGAGTGCAGTGGTGTGATCATGGCTCACTGCAGCCTGG
ACCTCCTGGGCTCAGGGGATCTGCCTACCTTAGCCTCTGTGTAGCTGGGACTACAGGCACGTGCCATCAG
ATCTGGCTAATTTTTTGTATTTTTTTTGTAGAGATGAGGTTTGGCCATGTTGCCCAGGCTGGTCTTGAAT

TCCTGGGCTCGAGTGTTCCACTCACCTGGGCCTCCCAAAGTGTTGGGATTACGGTTGTGAGCTCCTGTTC
CCAGCCTTGTTTTTTTTTTTATCTGTGGCTTGCTTGAGATGTAGAGAATTGCCCTTTGAGAAATTAAGAA
ATTACTTTTTTTTTTTTGAGATGAGTTTCACTCTGTCACCCAGGCTGGAGTGCAGTGGTGCAATCTCGGC
TCACTGCAACCTCTGCCTCCTGAGTCCAAGCGATTCTCCTGCCTCAGCCTCCTGAGTAGCTGGGATTACA
GGCATGTGCCACCATACCCAGATAATTTTTTTATTTTTAGTAGAGATGGGGTTTTACCATGTTGGCTAGG
CTGGTCTCAAACTCCTGGCCTTAATTGATCTGCCCGCCTTGGCCTCCTAAAGTGCTGCGATTACAGGCAT
GAGCCACCGCACCTGGCCTAAGAAATCACTTTTGAAGTGAATTCAAGGTATTACAGAACTGGGAAAAAGA
ACCAGTGAGGGGATACATAGGTTATACTGTTTTTGACTTTAAAAAATAATTTACGGGCCGGGCGCGGTGG
CACACGCCTGTAATCCCAGCACTTTGGGAGGCCAAGGCAGATAGATCTTGAGGTCAGGAGTTCGAGACCA
GCCTGGCCAACATGGTGAAACTCCGCCTTGCTAAAAATACAAAAATTAGCTGGGTGTGGAGGCGCAGCTA
ATGCCAGCTACTTGGGAGGCTGAGGCAGTAGAATCGCTTGAACCCAGGAAGCGGAGGTTGTGGTGAGCCA
AGATCAAAGTATACACAAGGTGGAGAAAGACCTATGTAACAGTAGTAAACCATGACACTCGAAAGAGGGC
TCAAGCTTGTGGTAGGCAATACTCGAAAACAGTGCATTCATTCATTCTCTGCTGTTGGAGGTACTTGAGT
GGAAATCCTGCAGCCCCTGACATCAGCACTAGCTAGCTTAATTTCCACTTCTGCACATACATTTATGTAG
TTGTTCTGGTTGGATGCCATTCTTAACTAATTTTTTTTTTTTTTTTTTTTTTGAGACCAAGTCTTGCTCC
GTCACCCAGGCTGGAGTACGGTGGTGCAATCACAGCTCACTGCAGCTTTGACCTCCTGGTCTCAAGTCAT
CTTCCAGCCTCAGCTGGGACTATAGGCATGTGCCATCATGCTTGGCTAATTTTTTAAATTTTTGTAGAGA
CAGGGTGTTGCCATGTTGCCCAAGCTGATCTTGAACTCCTGGGCTCAAGTGATCCTCCCACCTTGGCCTC
CCAAAGTGTTGGGATTACAGGCGTGAGCCACCGCGCTCAGCCGGATGCCATTCTTTACTCCCAAGTCCAA
CCTCTTATCTCACTTTTTCAGCAAAGCATTTCCTTAATGCTTCAATCAACATTCATCTTTCTTTTCAAAC
ATCCCTTATTATACGGTGCACACTGTGTGCTTGTGTTCTATGCTACGCACAGTAACTAGAATGTAGTAGA
TGTTTACTCAGATGTTTGTGGAATTTAAATCTCTGGTCTGCTGATGGCTTCCTGTTGAGATATCTTGGCT
TGTAGACTAGGTTAAAGCTTATTCTTCTCTCTCAAGTTAGTGCATTTCTGGGGACTTGTAAGATCCTTAA
CACTTCTTGATCTGAGACTGATAGACTCACAGGGAAAGGCTTTATAAACACCCTTTCTTCCTTCCAGTTA
CTCCTACCAAATATTTTGAGTCAATTTTTGTTCTTTCATTTCCCAAACCCACTCTATCTTGAATCCCATC
AGCCTTTAAAATACATCCAGTATTCCACTACTTCTCTCCAGGATTACTGCTACCACTTTGTTCCAAGTCA
CCACCACTGTCTCTTGTCTGGATTGCTAGAGTAGCCTCCTATTATAGGCTGAATAATCCTCTCCCCTGCT
GTCTGCCACAAGATGTCCATGTCTTAATTCCTGGAACCTGTGAATGTGTTACCTTTTGAGGTAAAAGAGA
CGTTGCAGATATGGTTAAGGATGGGTAGATTATTCTGGATTATCTAGGTGGGCCCAATATAATCACAAGG
GTCCTTAGAGAGGCAGGAGGATCAGGGTAGGTAGTAGGAGATGTGATGATGGAAGGAAGAGATTGGAGTG
ATGCTAGGAAGGGGTGTGCGTCCACGAGCCAAGGAATGCAGGTGGCCTGTAGAGGCTGGAAAAGACAGTC
TTCCCTCAGAGCTTCTAGAAGGAACCAGCCTTGTGCACACCTTGATTTTAGCCCGGAGAGACTGACTTTG
GTCTTCTAATTTACAGAACCGTAAGATTATAACTTTTTTTTTTTTTGTTAGTATTTATTAATCATTCTTG
GGTGTTCTTCGGAGAGGGGGATTTGGCAGGGTCATAGGACAATAGTGGAGGGAAGGTCAGCGGATAAACA
TGTGAACAAAGGTCTCTGGTTTTCCTAGGCAGAGGGCCCTGCCGCCTTCCGCAGTGTTTGTGTCCCTGGG
TACTTGAGATTAGGGAGTGGTGATGACTCTTAACGAGTATGCTGCCTTCAAGCATCTGTTTAACAAAGCA
CATCTTGCACCGCCCTTAATCCATTTAACCCTTAGTGGACACAGCACATGTTTCAGAGAGCATGGGGTTG
GGGGTAAGGTCATAGATAAACAGCATTCCAAGGCAGAAGAATTTCTCTTACTACAGAACAAAATGGAGTC
TCCTATGTCCACCTCTTTCTACACAGACACAGTAACAATCCGATCTCTCCTTCCTTTCCCCACATTTCCC
CCACTTCTATTCGACAAAACTGCCATCGTCATCATGGCCCGTTCTCAATGAGCTATTGGGTACACCTCCC
AGACGGGGTGGCGGCTGGGCAGAGGGGCTCCTCACTTCCCAGACAGGGCGGCTGCTGGGCGGAGGGGCTC
CTCACTTCTCAGAGAGGGCGGCTGGTCAGAGAGGCTCCTCACCTCCCAGACGGGATGGCGGCGGGGCAGA
GACACTCCTCAGTTCCCAGACGGCGTCGCGGCCGGGCAGAGGCGCTCTTCACATCTCAGACGGGGCGGCG
GGGCAGAGGCGCTCCCCACATCCCAGACGATGGGCAGCCGGGCAGAGACGCTCCTCACTTCCTAGACGGG
ATGACGGCCGGGAAGAGGCGCTCCTCACTTCCCAGACTGGGCGGCCGGGCAGAGGAGTTCCTCACATCCC
AGACGATGGGCGGCCAGGCAAAGACACTCCTCACTTCCTAGACGGGGTGGCGGCTGGGCAGAGGCTGCAA
TCTCAGCACTTTTGGGAGGTCAAGGCAGGCGGCTGGGAGGTGGAGGTTGTAGCGAGCCGAGATCACGCCA
CTGCACTCCAGCCTGGGCAACATTGAGCACTGAGTGAGCGACACTCCGTCTGCAATCCTGGCACCTCGGG
AGGCCGAGGCTGGCAGATCACTCATGGTCAGGAACTGGAGACCAGCCCGGCCAACACGGTGAAACCCCGT
CTCCACCAAAAAATACGAAAACCAGTCAGGCGTGGCGGCGCGCGCCTGCAATCCCAGGCACTTGGCAGGC
TGAAGCAGGAGAATCAGGCAGGGAGGTTGCAGTGAGTCGAGATGGCAGCAGTACAGTCCAGCCTTGGCTC
GGCATCAGAGGGAGACCGTGGAGAGGGAGAGGGGGAGGGGGAGGGGGAGAGGGAGAGCTGTTTTTTGTTT
TGAGACAGAGTCTTGCTCTGTCACCCAGGCTGGAGTGCAGTGGCACAATCTTGGCTCACTGCAGCCTCCG
CCTCCTGGGTTCAAGCAGTTCTCTGCCTCAGCCTCCCGAGTAGTTGGGATCACAGGCGCCCACCACCATG
CCCGGCTAATTTTTGTATTTTTAGTAGAGATGGGGTTTCACCATCTTGGCCAGGCAGGTCTTGCACTCCT
GACCTCGTGATCCACCCGCCTCAGCCTCCCAAAGTGCTGGGATTACAGGTGTGAGCCACTGCACCCGGCC
GTAAATTTGTGTTGTTTTAAGCCACTAAGTTTGTGGTAATTTGTTATAGCAGCAGTGGAAAACCAAGACA
CCTCCTAAATCATCTGCCATTACTGTTATATTGCTACCATTTGTTCTCAGGTTAGCAGCCAAAGTAGTTC
TTTAAAATCTAAGGCTCATCACATCACTCCACTAACTCAGAAACCTTCAGTGGCTATTTCATTAATAGAG
ATTTATAGCCAAAATCTGTTTTGTGACTGAGAAGACATGACAGGATTCTGTGCTCTCATCCCTTACTTTT
AAATTTGTTTTCCCCAAAATTCATAGGTTGAAACACTAGCCACCCAGTACTCAGAATGTGACTGCATATG
TATGTAGGGCCTTTAAATATGTGATTAAGTTAAAATGAGGTCTTTAGCGTAGACCCTAAAACAATTTGAT

GGGTGTCCTTAAGATTAGGACACAGAAAGGAGGCCAAGGCGGGCAGATCACCTGAGGTTGGGAGTTCGAG
ACCAGCCTGACCAACATGGTGAAACCCCTTCTCTACTAAAAATACAAAAAATTAGCTGGGCGTGGTGGTG
CATGCTTGTAATCCCAGCTACTCAGGAAGGCTGAGGCAAGAGAATCACTTGAACCTGGGCGGCAGAGGTT
GCAGTGAGCCATTGCACTCCAGCCTGGGCAACAAGCGTGAAACTCTGTCTCAGAAAAAAAAAAAAAAAAA
TTGGGACACAGAAGAAACACACAAAGACCATGTGAGGACACAGCAAGAAGGTGGCCATCTGCAAGCCAAG
GAAATAGGCCTCAGAAGAAACCGAATGCATCCACACCTTGATCTTGGACTTTTGGCCTACAGAACTATAA
GAAATAAAACTTTTTTTGTTTAAGCCATCCAGTCTTTGGTACTGTGTTATGGCAGTCTTGGTGAACTAAT
ACACTGTTGTCATTTTCAGTAACTGTCCATTATTGAAAATGGGATATTGAAGTCTCCATTATTGTTGAAC
TATGTATTTCAGTTCTGTCAAGATCATTTGATCATATTCATGAAGGTTTATTTCTGGGTTTTCTTATTTG
TTTCAAGGATCTGTGTGTCTGTTTTTTTTTTTTTTTCAGATGATGTTTTACTCTGTTGCCCAGGCTAGAG
TGTAGTATGTTCACAGCAACCTCTGCCTCCCAGGCTCAAGCAATTCTCCAACCTCAGGCCCCTTGAGTGG
CTGGGACTACAGATGTGCACTACCATGCCCAGCTAATTTTTTTTTTTTTTTTTTTGAGACAGAGTCTCGC
TCTGTTGCCCAGGCTGGAGTTCAGTGGCGCTATCTCGGCTCACTGCAACCTCTGCCTCCCGGGTTCAAGC
AATTCTTGTGCCTCAGCCTCCTGAGTAGGTGGGACTACAGGCATGCACCACCAACCCTGGCTAATTTTTT
GTGTTTTTAGTAGAGATGGGTTTTCGCCATGATGGCCAGGCTGGTCTCGAACTCCTAACCTCAAGTGATC
CTCCAGCCTGTGCCTCCCAATGTGCTGGGATTACAGGCGTGAGCCACTGCACCCGGCCTTTTTTAAAAAG
TTTTTTTGGTCAGCCTCTTGTAGTCTCTATTGATGTCATAGCCTCCGGCATCTGTGATGTTAAACAATTG
CCACAGATTTTTTTTTTGACACGTGGACACGTGCCCTCAGGATGGGGCTGTTGCATAGAGTGAACTTTGA
ATCAGGTCGAATAAAGACAAGCCTTCAGGATAGAGCTTTTTCGGAGCTGCCAGATAGGTCAAATATTGAC
AGTTCTCTGAGGATAGTGCTTTTTGGGAGCTACAAACTCGTTGTGTTCTCTCCAGTGGCTTCTAGCTTGC
TAGTTTCACATTGCAGTTGGAAGGGTGCTGGTTTTATTTTTTATTTTTTTTTCTCTCTCTCTTTTTCTCT
TTCTTTCTTTCTTTCGACGGAGTCTCCCTCTTGTCGCCCAGGCTGGAGTGCAATGGTGCGATCTTGGCTC
ACTGCAACCTCCATCTCCTGGGTTCAAGCGATTCTCCTGCCTCAGCCTCCTGAATAGCTGGGATTACAGG
CGTGTGCCACCACACCTGGCTAATTTTTTATATTTTTGGTACAGATGGTGTTTCACCATGTTGGGCAGGC
TGGTCTTGAACTCCTGACCTCAAGTGATCCGCCTGGCTCAGCTTCCCAGAGTGCTGGGATTACAGGTGTG
AGCCACCATGCCTGCCAGACATTTTTCTTGAATAAATACTCCTTGGACTTGGAAAGGCTTTGGTTTATCT
GCAGAGCTCTGAAAGAGTCAATGTTGATCATTTTTGGCAGTATTCTCATAGCTTTTATGGAGGAGCAGAT
TTCCAGAGGTCCTTCCTCTGCCATTCTGGAAGTTTCCTCTCCCCAGTAACATTTCTGGCTGTACCACTCC
CGGTTCTCCCATATCACTCTGCTTCAGCTGCTCTGGCTTCATTGTTTCTTGAATGTGCCAGGCAAGCTTC
TGTCATGTGACCTTTGCATTGCTTTTACCTCTACCGGAAATATTTTCCCCCAGGTGTTTGCCTGGTTTGC
ATCCTCATCTCCTTCGGGTCTTTGGTCTATATCATCTTCTCAGTGAGGCTTTTCCTGACCACTCTTGTTT
TTGGACAGTCTTATTTAGAATTCAACCCTCCTAACCCCATGTATTTCTAGTCTTCTTTTCAGACCATTTT
CTCCTTTTTTTTTTCTTTTGAGATGGAGTCTGTCTCTGTTGCCCAGGCTGGAGTGCAGTGGCACGATCTT
GGCTCTCCTCGGCTCACCTTGGCTCACTGTGCCACTGCACCACTACGCCCGGCTAATTTTGTATTTTTAG
TAGAGACAGGGTTTCTCCATGTTGGTCAGGCTGGTCTCGAACTCCTGACCTCAGGTGATCCACCTGTCTT
GGCCTCCCAAAGTGCTGGGATTACAGGCGTGAGCCACTGTGCCTGGCCCCAGTTATATTTTTTTAAAAAT
TATTATTTTTTTGTTTTTGTTTTTGTTTTTTTTAAGACAAGGTCTCACTCTGTTGCCCAAGCTGGATGGA
GTGCAGTGGTGTGATCACAGCTCACTGCAGCCTCAACCAGCGGGGCTGAAGTGATCCTCATGCCTCAGCT
TCCTGAATAGCTGGGACTACAGGCATGCCTCACCATGCCTGGCTAATTTTTTTTTTTTTTTTTTTCTGTA
AGTATTTTATTTATCACTGAAGAAAAAACACAGCAGCAAGTTCTGTGTTGGCTTCAATGAGACCAAATAG
TTCATCTTTAACATAGCTACTGCGCTCCAAAATTGAAGTCAACACAGTCATTACTACAAATTACTTGTTG
AAAGAATCTATCTTCAAAAGAAAGAAAATTAGGAAAGAGAATTTAAACGATTCCTCCAAACCATACATTT
ATAAATATTGTCATATTTAAAATGCTTGAAAGGCGTGAATTCTCCATTAATGGAAAAGCAGTTTGCTATT
GGCAACAGAAAAAAACTCAGCGATAATTTACCAAATGCTTTAAAATTTAAAAGCTATACTGTTTGGATTC
ACACTTGCGCAGTAATCTTGTCATTACAACAACTGCAAATTGCTTTGTACTCACCTTCTCTCAAGTAAAA
TCTAAAGAAAACCCCACCGTATCCAATGTTTGTAATGTTTACTTCAAGTGAATCTAAAAAATTTCTAAAT
TTAGTTTATGGTAAATACCTCAAAATACCTAAAAATAGGTACGTATCAACCACTTAAGGGTGTGAAAATT
GTTTCAGATTCACTGCTTTAAAGGGTCTTCAAATATATTTTCTTTAACAGAACCTTACTTCATTCCAAAT
TATTATCAGATATTTCAAAATAAAAAAAAAGTATCAGTTCTATACCAAGATAAAGGTGTTACTCAATACC
TGAAGGTTCACATTTTAAATTTATTTTTTAGTTTTTCTTTTTTGTCAACAAATGATTGATGAAATAATGC
AACACCCTTAAATTCCAGAAAATGGCATGGTTTTTCCAACCTCCTGTGGATATGGTGCATGATCAATCTA
TTATATAGGAGCCTGGCTAATTTTTAATTTTTTTTTTTTTTTTTTTGAGAGGGAGTTTCACTCTGTCACC
AAGGCTAGAGTGCAGTGGCACGATCTCGGCTCACTGCAACCTCTACCTCCCAGGTTCAAGCGATTCTCCT
GCCTCCACCTCCCAAGTAGCTGGTATTACAGGCATGCGCCACCATGCCCAGTTAATTTTTGTATTTTTAG
TAGAGGTGGGGTTTCGCTATGTTGGCCAGGCTGGTCTCAAACTCCTGACCTCAAGCGATCTGCCCGCCTC
AGCCTCCCAAAGTGCTGGGATTACAGGCATGAACCACTGCGCCTGGCTAATTTTTACATTTTGTGTACAG
ACAGAGTCTTGCTATGTTGCCCAGGCTAGTCTTGAACTCCTGGCCTCAAGGTATCCTCCTGTCTTACTCT
GTCACCCAGGCTGGAGTATATTGGCTGGCTGTTCACAGGTGCAACCATAATGTACTACAGCCTTGTGCCC
CTGGGCTTAAGTGATCCTCCCACCTCAGCCTCTTGAGTAGCTGTGACTACAGGTGCATGCTGCCTCACCT
GGGTGCTGTTTTTTAGTAGTATTTATTCTGTTTTTTCATTTATTATTATTATTTTTTTGAGATGGAGTCT
TGCTCTGTCATCCAGGGTGTAGTGCAGTGGTGTGATCTTGACTTACTGCAACCTCTGTCTCCTGGGTCAA
GCAATTCTCATGCCTCAGCCACCTGAGTAGCTGGGATTACAGGCATGCACCACCACACCTGGATAATTTT

TGTATTTTTAGTAGAGATGGGGTTTCACCATGTTGGCCAGGCGGTCTCAAACTACTGACCTCAAGTGATG
CTGCCCCACTTTGCCTCCCAAAGTGCTAGGATTACAGGCATGAGCCACCATACCCAGCAGTGTATTTATT
CTAATACGATTAGTTGTATCATGAAACAATTAGAGTGCTCTCTGTTTATATTGTGTATATAGTTTCATCT
CACCGCTGCCACCCCGCCCCCTCCCAGGGAGACTTGCTTGAGTGAGGTTGCAGCTCTGGCAGGAGCAGTT
GGGGAATATGTTTTTTATAGCATCATTAGTAGCTTATTTAATTGACTGTTATTTTCTCATGTTGACCATT
AGCCAAAGATCACTCTTCTTTGGATATGAGATATCCAGGCTATTATTTTTCTGAAGAATTCAGTGTTTCC
ATCCTACTTTTTCCAGTCTCCTAAAGACTGGGACCCAAATGCTATGGTTAGAAGGTATTTAGTTTCTCAG
TTTTGGCATAGATATCGAATCAGTTTTTCCAATAAAAATGTGTTTAACATTTTGTACACTTATACTACTA
CTATGCTTTTAGTAGGTAATACTGAAATGGTTATAATACTGTCCCTTGTTCTGTAAAAAAATATAGTACA
TTTTGAGAGAAAAACTTGATGTGAAAGTTTCCCTTCCATGATCAGTGATAGGGATCTTTCCTTGGCTCTT
AATTTCTTATTTTCATATACTTATCTCTGCATGTGCCTTATTACTTATCCCTAACTATAAGCCCCTTAAG
GGAATAAGAGATAAACCCCGTATTACTTATCCCAAACTTAAGCCTGTTTTCCATCTTTGTACTATTTGCA
ACATGAAAAATTGTGTTTGAATTACTGAATGCACTAATTTATGTGAATGTTGTTTTAAATCCATAAAATG
CAGAGAAGTGTTGGGTGATATTTTTCTTTTTGGTCAAGTGATGTTTTCTAAAAAATAATAGAAAGCATCT
AATTGTTGAATGAGTGGCATTGGTGCTAAGTTTTATGAGGCTTCTGGAGATGATATAACTGTGGACTGGG
AAGTTGAGCAAGACTTTTCAGAGAAGGTGGGATTCAAGTTCTGTTAAGGAAGAATAGATGTTCCATGGTT
AGGAATGGGAAGTGTATTCAGGTTGGAGGGAAGAGCTTGTGCACTGGTGGGGATACAGCATGTAGGGCAA
ATTTAGAGACCAGTGAATGGGTCAGGTGTGCTCCAGTGGAGACTGTGCATGTGTGGGTGATTGACAGCAG
AGTGGGCAAGTTGTGATTGAGCCTTATACTTGCCATGTGGCCTTTAGACAAAGTATTTTACTTCTACTTT
TTTTTTTTTTTTTTTTTGAGATGGAGTCTCGCTCTGTTGCCCAGGCTGGAGTGCAGCGGTGTGATCTCGG
CTCACTGCAACCTCCGCTTTCTGGGTTGAAGCGATTCTCCTGCCTCAGCCTCCTGAATAGCTGGGATTAC
AGGCGTGCGCCACCAGGCCCAGCTAATTTTTGTATTTTTAGTAGAGACGGGGTTTCACCATGTTGGTCAG
GCTGGTCTCAAACTCCTGACCTAGTGATCTGCCTGCCTTGGCCTTCCAAAGAGCTAGGATTACAGGCATG
AAACCACCGCGCCTGGCCTTACTTCTACTTTTTATTTTTCCTTTTTCTTCTTCTTTCTTTCTTCCTTAGT
TTCTTTTCTTTTTTTTTTTCTTTTTTTTTTTTTAAGAGACACTGTCACTGTCTGGCCCAGGCTGGAGTGC
AGCGGCATTATCACAGTTCACTGCGGCCTTGAATTCCGGGGCTCAAGCAGTCCTCCTGCCTCAGCCTCCC
AAGTAGCTGGGACCACAGGCATACACCACTATGCCTGGTTAATTTTTGTATTTTTAGTAAAGCTGGGGTT
TTGCCATGTTGGCCAGGCTGGTCTTGAACTCCTGACCTCAGGTGACCTGCCCACCTCTGCCTCCCAAAGT
GTGGGGATTATAGGCGTGAGCCACTGCACCTAGATGTCAGTTTGATTCTTATGACGCCACTTCCCTTCTT
TATACATCCCCTTCCCCACATCTTGTTTGGTATTTAATGGATTTCTCTTCTTTTCCAAAGGATCTACCGC
TACCAGTCTCATGACTATGCCTTCAGTAGTGTCGAAAAATTACTTCATGCTCTAGGAGGAGATGACTTCC
TTGGAATGCTTAATCGAACACTTCTTGAAACCTTGCAAAAGGCCGGCTTTTCTGAGAAGTTCCTCAATGA
AATGATTGCTCCTGTTATGAGGGTCAATTATGGCCAAAGCACGGACATCAATGCCTTTGTGGGTAAGCCA
GGGCTTGAAACAGTGGTGGACATTAGTTCACAGAGGGGCTTACCTGATGCTATGGTGGTTCCTGACTTTA
GGCTGTGGATTGGAGGCTACTTTTCTCTGTGAACCTCAGACATTGTTAGCAAAATTGTGCGTATGTAGAT
GTGCATTTTTAGGGGGAGAGGGTCTTTAGCTTTCATTAGTTCAGTAATTCTTAACTGGGGGAGATTTTGC
TCCCAGGGGCCATTTAGCAATGTCTAGAGACATTTGGAGTAGAACATGTAATTGGGAAGGGAGAGTGGGT
AGAGAGCCTCTTACAAAAAGTCAGAATTAAAACACATTTTTCCTCCTATTGACAGGGGCGGTGTCACTGT
CCTGTTCTGATTCTGGCCTTTGGGCAGTAGAAGGTGGCAATAAACTTGTTTGCTCAGGGCTTCTGCAGGC
ATCCAAAAGCAATCTTATATCTGGCTCAGTAATGTACATCGAGGAGAAAACAAAGACCAAGTACACAGGT
AAGCTTGAATTTCTTATTGTTCCTGATATCCCCTGCAGAAAATGATTGCTCAGGGAGAAACACTTCTCTT
CTGTCATCTCTTACTCAGTTCTAAAATGATGAAACTTGAAATTGTATATATTGTACCATATAGAAATATT
CCCCAATGGGCCAGGCACGGTGGCTCATGCCTGTAATCCCTGTACTTTGGGAGGCCAAGGAGGGTGGATC
ACCTGAGGTCAGGAGATCAAGACTAGCCTGGCCAACATAGTTAAACCCTGTCTCTACTAAAAATACAAAA
AATTAGCCGGGCGTGGTGGCAGGTGCCTGTAATCCCAGCTACTCGGGAGGCTGAGGCAGGAGAATCACAT
GAACCCAGGGGGCAGAGGTTGCAGTGAGCCAAGATCACACCACTGCACTCTAGCCTGGGTGACGGAGTGA
GACTCCATCTAAAAAAAAAAAAAAAAAAAAAAAATTCCCCAGTGTAGTTAAGTGCTGTGGCATCACTCCT
TGGATTTGAAAAATAATCTTTTTTTTTTTTGAGATGGAGTCTCGCTCTTTTGCCCAGGCCGGACTGTAGT
GGCCTATCTCGGCTCACTGCAAGCTCCGCCTCCCGGGTTCACGCCATTCTCCTGCCTCAGCGTCCCGAGT
AGCTGGGACTATAGGCGCCTGCCTCAGCGTCCCGAGTAGCTGGGACTATAGGCGCCTGCCACCGCGCTCG
GCTAATTTTTTGTATTTTTAGTAGAGACGGGGTTTCACCATGTTAGCCAGGATGGTCTCGATCTCCTGAC
CTCGTGATCCACCCACCTCGGCCTCCCAAAGTGCTGAGATTACAGGTGTGAGCCACCATGCCCGGCCGAA
AGATAATCTTACCTTCAAAAGTAAAAATTCTGTATTTTGTACTTACTGACATTCTGAATAAGAAATTGGC
CTCAATGTAATAAAAATGCAAGGGACAACTATCTTAAGGGATATAACTTCTTCAATTCTTTAGAATTATA
GAGAAATTATGAAGAAGGTATACAATTATTTTTAGAAGGTATGTTAGAAACACTAAACAAATATATGCCT
CCTTATTTCCTCTAGGAAATCCAACAAAGATGTATGAAGTGGTCTACCAAATTGGAACTGAGACTCGTTC
AGACTTCTATGACATCGTCTTGGTGGCCACTCCGTTGAATCGAAAAATGTCGAATATTACTTTTCTCAAC
TTTGATCCTCCAATTGAGGAATTCCATCAATATTATCAACATATAGTGACAACTTTAGTTAAGGGGGAAT
TGAATACATCTATCTTTAGCTCTAGACCCATAGATAAATTTGGCCTTAATACAGTTTTAACCACTGATAA
TTCAGATTTGTTCATTAACAGTATTGGGATTGTGCCCTCTGTGAGAGAAAAGGAAGATCCTGAGCCATCA
ACAGATGGAACATATGTTTGGAAGATCTTTTCCCAAGAAACTCTTACTAAAGCACAAATTTTAAAGCTCT
TTCTGTCCTATGATTATGCTGTGAAGAAGCCATGGCTTGCATATCCTCACTATAAGCCCCCGGAGAAATG

CCCCTCTATCATTCTCCATGATCGACTTTATTACCTCAATGGCATAGAGTGTGCAGCAAGTGCCATGGAG
ATGAGTGCCATTGCAGCCCACAACGCTGCACTCCTTGCCTATCACCGCTGGAACGGGCACACAGACATGA
TTGATCAGGATGGCTTATATGAGAAACTTAAAACTGAACTATGAAGTGACACACTCCTTTTTCCCCTCCT
AGTTCCAAATGACTATCAGTGGCAAAAAAGAACAAAATCTGAGCAGAGATGATTTTGAACCAGATATTTT
GCCATTATCATTGTTTAATAAAAGTAATCCCTGCTGGTCATAGGAAAACACACGGTTCTAATTAAGTGTG
AAGGTATAGCTATTGCACTTATGCCATCTCCAAAATTTCTTAAGTATTCTTTCACTATCCATTAGGAGTT
TTTCTTAAACTTGTCTGATAATAAGAATCACCTGGAGTTAGGAGGTGGTGGTTGCAGTGAGCCAATCTCA
CCATTGCACTTCAGCCTGAGCAACACGAGCAAAACTCCGTCTCAAAAGTAAATAAAAATAATCACCTGGA
GTTTGTTAAACCATATGGATTCTCAAGCTCCTCTCTTGAAGATTCTGATTCAGTAGGTCTGGGAGTGGCG
CGCTGGATTTTGATCAAAATTGTAGAGCATTTTAAGGTGAGTACCTGAGGGAGAACTTAAAGACATCTTA
GTTGGGGAGTAGTCCTTTTGAATTTTACAGCTAGATATAATCTTCAGTCAGATAAAATTTATGGGAGCTG
GTGTCTTATGCCTGACTCTTAGTAATTTCATACCGGTTTGAAGTACGTGTGCCCATGCCTAAAGCCTTGA
CTTTCAGAATGTTGTCTTTTGATTCTTCTGTCTTGATTTGATTAGGGGTGAAATTTAGAAGTCTTAGTAA
TGTAACTTGAAGATGTTAAACAAAAATCTCAAGTAAAATGAAAAGCAAATATGGGCTACTGAATTAAGAA
ACTGGCATTCTAGTATTAAATCCTCACTTCAGGAGCTTTTAAAAATACTGAGACCCCCCCATAACCAGAG
ATTCAGATTCAAAGACTGAGGATAGGACCTTAGCATTGTAGCTATTTAAAGTTTCTAATGTGCACCCAGG
GTTGGGAATCACCAATGTGGGTGTGAAAATGCCTACAAAGGGTTTTAGTGCCTTAGAAGTCCTAAGAAGC
CCAATCTGTATCAAAGCAGATCCATTTTGCAAGGATCTTTCTTTTAGAACTTTCTCAGTTCTCTTAGTAA
GAACTTTAGAAGTAATCTTGATAATAAGCACAGACAGCCTAACAGCAGAGGCAACTTAAATAACTCCTGA
GCAGTTGGCACTAGAACAGAATACTTGGAATGACACCAAAGTTAACCAAGTCCAGCATATGTCCAAAGAG
TTAAGTGTTTCATTTACTGTAGCATTCTGGGTGAGAAATTGGTTGCTGAAATCTTAAGACAGTGGTCTCA
ACCTTGGCTGCACATTGGAATCACCTGTAGGGTTTTAAAGCATCCAAATGGTAATTAACAGGCAGCAAAA
CTTCAGAACTAGTTCTGCATCTACTGTGCAAAGATCATGATTAACTGTCAAGACACTGGTAGAACAGAAC
AAGCAAAAGATTAAGAGTTCAAAAGTAAATGCAACCAATTTAACATGTAGTGTTATTAAAAAATTACAAA
GGCCTAGACCAGCCTGGGCAACATGGTGAAACCCCATCTCTACAAAAAATTTTTAAAAAGTTGGCCAGGC
ATGGTAATGCGCGCCTGTGGTCCCAGCTGCTCGGGAGGCTGAGGTGGGAGGATCACTTGAGCCTTGGAGG
TCAAGGCTGCAGTGAATCATGATCATGCCACTGTACTCCAGCCTGGGCAACAGAGACCATCTTAAAAAAA
AAAAATCCTTCCATTGAATAGAAACTTCAAAGTCCTTCATCAGATCATGGCATTAGCTACTTTAGCAAAA
TAGTGTAAACTTTGGTTCTGAGTAAAAATGACTTCTTCCCTACCACAGTTGTGAATGTTAATATGCTGAT
AAAACTGCCAAGATATATTTGATAGGTAGGGGATTAAACTCATGGTTTGTCAAAAGAGTGTTTTTTTCTA
GTTTTATTCTTAACAGATATGTTGAGGTATTCATATTTGTTTCCTTTTGTGGTTTTAATGAAGACAATTT
GTAAAGTAATACTGTTATGTATATGCTAAATGTTGGTAAATACTAATTAATTTCCATCATTTGTAGACTT
GTTTTGCAATGGGATATATTTTTACTTATAATACCAATTTAGGCTGGGCGCAGTGGCTCACGCCTGTAAT
CCCAGCACTTTGGGAGGCCAAGGCAAACGGATCACGAGGTCAGGAGATCAAGACCATCCTGGCCAACATG
GTGAAACCCCATCTCTACTGAATACACAAAAATTGGCTGGACATGGTGGCGCATGTCTGTAATCCCAGCT
ACTGTAATCTCAGCTACTTGGGAGGCTGAGGCAGGAGAATTGCCTCAACCGGGAGGCGGAGGTTGCAGTG
AGCCAAGATCGCACCACTGCACTCCAGCCTGGCAACAGAGCGAGACTCTGTCTCAGAAAAAAAAAACAAA
AACCAGTTTAGGCTGGGTATGGTGGCTCACCAGCACTTTGGGAGGCTGAGGCAGGTGGATCTCTTGAGGT
CGGGAGTTTGAGACCAGCCTGGCCAACATGGTGAAACCCTGTCTTTACTAAAAATACAAAAATTAGCCAG
GCATGGTGGCGTGTGCCAGTAATCCCAGCTACTTGGGAGGCTGAGGCGCGAGAATCGCTTGAACCCTGGA
GATGGAGGTTGCAGTGAGCAGAGATCGCACCACTGCACTCCAGCCTGGGCAAGAGTGAGACTGTGTCTCA
AAAGGAAAAAAAAAAAATTAAAAATGTTGCTGGTATTACATCAAACCTACTCTGGATAAATTTTCCAGTA
GGGCATCTGAAATTGCTTTGGCAAAAGCAAAACAGGTTATCAATACACCAAGGACATCAAGTAGTTTTAA
TGCAACTACAGTGTTGAGTGCTTTTTTACTGGGAGAAAAATCCTAGCAGAACCTGCTACTCTGGGCGCAT
TAGGTAGTTTTATGCAACTATAGTATTAAGAGTTTTATTGGAAGAAAAAACACTGTCAGAGCTTACTGCT
CCAGACCTTTTTTTTTAATAGAAGAGACTGGTAAAAATGATGCAGGGGCTGAATGAAAGCCAGCTCTGCT
CCAGTGGCTCTCTGAGTTGAGCATGCATCAGTTACCTTAAGGGCTTGTTAAAACATCGCTAGGCTCCACC
CTGTTTATTCAATAAGTTTGTTATGGGATATAAGAAGATGCATTTTATTAAGTTCCCCAGTGATGCTGCT
GTTCTACTATAGGAACCACACTTGAAGAACTAGTTCTACAATTCAGCTGTATGTTGTAAAGGAGTCAAGT
CAATGAAGGAGTTATAAAGAGTTGCTGAAAAATGGAGTAACAGTGTATGGGACTTCACATCAGTTCTCCT
TATTGATTGTTAGTTTGATCCCTCTTGTTCTTTTGTTGTAAACATTTTCTATAATTAGGAAATGCCATTT
AAGAGTGAGAGAGGTATATATCTATGAGCCATTGTGTTTGGTGTTTTACAAGAACTTTACCATACTGGTG
TGTAGTCCATTCTGTACAGTTTAAAAGTGATTCACGATTTGCAGGCTTTTTATCAGATCACAAAAAAATC
AGTCTTTAAGCATTTGCTTGGTAAGGTTTCTTAAGATTAGGTTTATAATACAACCATCTGTAATGTATCT
CTCGTTTGAGCTTGTGGGCCATACAATTCATTAACTAGATGAATACATTGTGGACAGCATCCTCACTACC
CCTCTCTACTCACTCACAAAGAACCATGATACACTGGAATGTTTTTCTCTGGAATCCTCTTTCTACTCTT
GTATTAAAATTTTTCCCCACTTCATGATTATATTCAAAATAAACAACTACATCATTA

(SEQ ID NO:1).

The mRNA sequence of PCYOX1 is:
>gi|166795300|ref|NM_016297.3| Homo sapiens prenylcysteine oxidase 1 (PCYOX1), mRNA

GCGCAGCCGCGCAGCGGTGGGAGGACTGCGGGGCTCTTGAGGCCAGCTGCAGAGCTTGTGGAGGCCATGG
GGCGCGTCGTCGCGGAGCTCGTCTCCTCGCTGCTGGGGTTGTGGCTGTTGCTGTGCAGCTGCGGATGCCC
CGAGGGCGCCGAGCTGCGTGCTCCGCCAGATAAAATCGCGATTATTGGAGCCGGAATTGGTGGCACTTCA
GCAGCCTATTACCTGCGGCAGAAATTTGGGAAAGATGTGAAGATAGACCTGTTTGAAAGAGAAGAGGTCG
GGGGCCGCCTGGCTACCATGATGGTGCAGGGGCAAGAATACGAGGCAGGAGGTTCTGTCATCCATCCTTT
AAATCTGCACATGAAACGTTTTGTCAAAGACCTGGGTCTCTCTGCTGTTCAGGCCTCTGGTGGCCTACTG
GGGATATATAATGGAGAGACTCTGGTATTTGAGGAGAGCAACTGGTTCATAATTAACGTGATTAAATTAG
TTTGGCGCTATGGATTTCAATCCCTCCGTATGCACATGTGGGTAGAGGACGTGTTAGACAAGTTCATGAG
GATCTACCGCTACCAGTCTCATGACTATGCCTTCAGTAGTGTCGAAAAATTACTTCATGCTCTAGGAGGA
GATGACTTCCTTGGAATGCTTAATCGAACACTTCTTGAAACCTTGCAAAAGGCCGGCTTTTCTGAGAAGT
TCCTCAATGAAATGATTGCTCCTGTTATGAGGGTCAATTATGGCCAAAGCACGGACATCAATGCCTTTGT
GGGGGCGGTGTCACTGTCCTGTTCTGATTCTGGCCTTTGGGCAGTAGAAGGTGGCAATAAACTTGTTTGC
TCAGGGCTTCTGCAGGCATCCAAAAGCAATCTTATATCTGGCTCAGTAATGTACATCGAGGAGAAAACAA
AGACCAAGTACACAGGAAATCCAACAAAGATGTATGAAGTGGTCTACCAAATTGGAACTGAGACTCGTTC
AGACTTCTATGACATCGTCTTGGTGGCCACTCCGTTGAATCGAAAAATGTCGAATATTACTTTTCTCAAC
TTTGATCCTCCAATTGAGGAATTCCATCAATATTATCAACATATAGTGACAACTTTAGTTAAGGGGGAAT
TGAATACATCTATCTTTAGCTCTAGACCCATAGATAAATTTGGCCTTAATACAGTTTTAACCACTGATAA
TTCAGATTTGTTCATTAACAGTATTGGGATTGTGCCCTCTGTGAGAGAAAAGGAAGATCCTGAGCCATCA
ACAGATGGAACATATGTTTGGAAGATCTTTTCCCAAGAAACTCTTACTAAAGCACAAATTTTAAAGCTCT
TTCTGTCCTATGATTATGCTGTGAAGAAGCCATGGCTTGCATATCCTCACTATAAGCCCCCGGAGAAATG
CCCCTCTATCATTCTCCATGATCGACTTTATTACCTCAATGGCATAGAGTGTGCAGCAAGTGCCATGGAG
ATGAGTGCCATTGCAGCCCACAACGCTGCACTCCTTGCCTATCACCGCTGGAACGGGCACACAGACATGA
TTGATCAGGATGGCTTATATGAGAAACTTAAAACTGAACTATGAAGTGACACACTCCTTTTTCCCCTCCT
AGTTCCAAATGACTATCAGTGGCAAAAAAGAACAAAATCTGAGCAGAGATGATTTTGAACCAGATATTTT
GCCATTATCATTGTTTAATAAAAGTAATCCCTGCTGGTCATAGGAAAACACACGGTTCTAATTAAGTGTG
AAGGTATAGCTATTGCACTTATGCCATCTCCAAAATTTCTTAAGTATTCTTTCACTATCCATTAGGAGTT
TTTCTTAAACTTGTCTGATAATAAGAATCACCTGGAGTTAGGAGGTGGTGGTTGCAGTGAGCCAATCTCA
CCATTGCACTTCAGCCTGAGCAACACGAGCAAAACTCCGTCTCAAAAGTAAATAAAAATAATCACCTGGA
GTTTGTTAAACCATATGGATTCTCAAGCTCCTCTCTTGAAGATTCTGATTCAGTAGGTCTGGGAGTGGCG
CGCTGGATTTTGATCAAAATTGTAGAGCATTTTAAGGTGAGTACCTGAGGGAGAACTTAAAGACATCTTA
GTTGGGGAGTAGTCCTTTTGAATTTTACAGCTAGATATAATCTTCAGTCAGATAAAATTTATGGGAGCTG
GTGTCTTATGCCTGACTCTTAGTAATTTCATACCGGTTTGAAGTACGTGTGCCCATGCCTAAAGCCTTGA
CTTTCAGAATGTTGTCTTTTGATTCTTCTGTCTTGATTTGATTAGGGGTGAAATTTAGAAGTCTTAGTAA
TGTAACTTGAAGATGTTAAACAAAAATCTCAAGTAAAATGAAAAGCAAATATGGGCTACTGAATTAAGAA
ACTGGCATTCTAGTATTAAATCCTCACTTCAGGAGCTTTTAAAAATACTGAGACCCCCCCATAACCAGAG
ATTCAGATTCAAAGACTGAGGATAGGACCTTAGCATTGTAGCTATTTAAAGTTTCTAATGTGCACCCAGG
GTTGGGAATCACCAATGTGGGTGTGAAAATGCCTACAAAGGGTTTTAGTGCCTTAGAAGTCCTAAGAAGC
CCAATCTGTATCAAAGCAGATCCATTTTGCAAGGATCTTTCTTTTAGAACTTTCTCAGTTCTCTTAGTAA
GAACTTTAGAAGTAATCTTGATAATAAGCACAGACAGCCTAACAGCAGAGGCAACTTAAATAACTCCTGA
GCAGTTGGCACTAGAACAGAATACTTGGAATGACACCAAAGTTAACCAAGTCCAGCATATGTCCAAAGAG
TTAAGTGTTTCATTTACTGTAGCATTCTGGGTGAGAAATTGGTTGCTGAAATCTTAAGACAGTGGTCTCA
ACCTTGGCTGCACATTGGAATCACCTGTAGGGTTTTAAAGCATCCAAATGGTAATTAACAGGCAGCAAAA
CTTCAGAACTAGTTCTGCATCTACTGTGCAAAGATCATGATTAACTGTCAAGACACTGGTAGAACAGAAC
AAGCAAAAGATTAAGAGTTCAAAAGTAAATGCAACCAATTTAACATGTAGTGTTATTAAAAAATTACAAA
GGCCTAGACCAGCCTGGGCAACATGGTGAAACCCCATCTCTACAAAAAATTTTTAAAAAGTTGGCCAGGC
ATGGTAATGCGCGCCTGTGGTCCCAGCTGCTCGGGAGGCTGAGGTGGGAGGATCACTTGAGCCTTGGAGG
TCAAGGCTGCAGTGAATCATGATCATGCCACTGTACTCCAGCCTGGGCAACAGAGACCATCTTAAAAAAA
AAAAATCCTTCCATTGAATAGAAACTTCAAAGTCCTTCATCAGATCATGGCATTAGCTACTTTAGCAAAA
TAGTGTAAACTTTGGTTCTGAGTAAAAATGACTTCTTCCCTACCACAGTTGTGAATGTTAATATGCTGAT
AAAACTGCCAAGATATATTTGATAGGTAGGGGATTAAACTCATGGTTTGTCAAAAGAGTGTTTTTTTCTA
GTTTTATTCTTAACAGATATGTTGAGGTATTCATATTTGTTTCCTTTTGTGGTTTTAATGAAGACAATTT
GTAAAGTAATACTGTTATGTATATGCTAAATGTTGGTAAATACTAATTAATTTCCATCATTTGTAGACTT
GTTTTGCAATGGGATATATTTTTACTTATAATACCAATTTAGGCTGGGCGCAGTGGCTCACGCCTGTAAT

CCCAGCACTTTGGGAGGCCAAGGCAAACGGATCACGAGGTCAGGAGATCAAGACCATCCTGGCCAACATG
GTGAAACCCCATCTCTACTGAATACACAAAAATTGGCTGGACATGGTGGCGCATGTCTGTAATCCCAGCT
ACTGTAATCTCAGCTACTTGGGAGGCTGAGGCAGGAGAATTGCCTCAACCGGGAGGCGGAGGTTGCAGTG
AGCCAAGATCGCACCACTGCACTCCAGCCTGGCAACAGAGCGAGACTCTGTCTCAGAAAAAAAAAACAAA
AACCAGTTTAGGCTGGGTATGGTGGCTCACCAGCACTTTGGGAGGCTGAGGCAGGTGGATCTCTTGAGGT
CGGGAGTTTGAGACCAGCCTGGCCAACATGGTGAAACCCTGTCTTTACTAAAAATACAAAAATTAGCCAG
GCATGGTGGCGTGTGCCAGTAATCCCAGCTACTTGGGAGGCTGAGGCGCGAGAATCGCTTGAACCCTGGA
GATGGAGGTTGCAGTGAGCAGAGATCGCACCACTGCACTCCAGCCTGGGCAAGAGTGAGACTGTGTCTCA
AAAGGAAAAAAAAAAAATTAAAAATGTTGCTGGTATTACATCAAACCTACTCTGGATAAATTTTCCAGTA
GGGCATCTGAAATTGCTTTGGCAAAAGCAAAACAGGTTATCAATACACCAAGGACATCAAGTAGTTTTAA
TGCAACTACAGTGTTGAGTGCTTTTTTACTGGGAGAAAAATCCTAGCAGAACCTGCTACTCTGGGCGCAT
TAGGTAGTTTTATGCAACTATAGTATTAAGAGTTTTATTGGAAGAAAAAACACTGTCAGAGCTTACTGCT
CCAGACCTTTTTTTTTAATAGAAGAGACTGGTAAAAATGATGCAGGGGCTGAATGAAAGCCAGCTCTGCT
CCAGTGGCTCTCTGAGTTGAGCATGCATCAGTTACCTTAAGGGCTTGTTAAAACATCGCTAGGCTCCACC
CTGTTTATTCAATAAGTTTGTTATGGGATATAAGAAGATGCATTTTATTAAGTTCCCCAGTGATGCTGCT
GTTCTACTATAGGAACCACACTTGAAGAACTAGTTCTACAATTCAGCTGTATGTTGTAAAGGAGTCAAGT
CAATGAAGGAGTTATAAAGAGTTGCTGAAAAATGGAGTAACAGTGTATGGGACTTCACATCAGTTCTCCT
TATTGATTGTTAGTTTGATCCCTCTTGTTCTTTTGTTGTAAACATTTTCTATAATTAGGAAATGCCATTT
AAGAGTGAGAGAGGTATATATCTATGAGCCATTGTGTTTGGTGTTTTACAAGAACTTTACCATACTGGTG
TGTAGTCCATTCTGTACAGTTTAAAAGTGATTCACGATTTGCAGGCTTTTTATCAGATCACAAAAAAATC
AGTCTTTAAGCATTTGCTTGGTAAGGTTTCTTAAGATTAGGTTTATAATACAACCATCTGTAATGTATCT
CTCGTTTGAGCTTGTGGGCCATACAATTCATTAACTAGATGAATACATTGTGGACAGCATCCTCACTACC
CCTCTCTACTCACTCACAAAGAACCATGATACACTGGAATGTTTTTCTCTGGAATCCTCTTTCTACTCTT
GTATTAAAATTTTTCCCCACTTCATGATTATATTCAAAATAAACAACTACATCATTA

(NCBI Accession no.: NM_016297.3; SEQ ID NO:2)

The amino acid sequence of PCYOX1 is: 
ORIGIN
        1 mgrvvaelvs sllglwlllc scgcpegael rappdkiaii gagiggtsaa yylrqkfgkd
       61 vkidlferee vggrlatmmv qgqeyeaggs vihplnlhmk rfvkdlglsa vqasggllgi
      121 yngetlvfee snwfiinvik lvwrygfqsl rmhmwvedvl dkfmriyryq shdyafssve
      181 kllhalggdd flgmlnrtll etlqkagfse kflnemiapv mrvnygqstd inafvgavsl
      241 scsdsglwav eggnklvcsg llqasksnli sgsvmyieek tktkytgnpt kmyevvyqig
      301 tetrsdfydi vlvatplnrk msnitflnfd ppieefhqyy qhivttlvkg elntsifssr
      361 pidkfglntv lttdnsdlfi nsigivpsvr ekedpepstd gtyvwkifsq etltkaqilk
      421 lflsydyavk kpwlayphyk ppekcpsiil hdrlyylngi ecaasamems aiaahnaall
      481 ayhrwnghtd midqdglyek lktel
//
 (NCBI PROTEIN ACCESSION GI:166795301, NP_057381.3; SEQ ID NO:3)

The invention will be now illustrated by means of non-limiting examples referring to the following figures.

**Figure 1****. PCYOX1** is associated **to lipoprotein.** (A) Immunodetection of PCYOX1 in lipoprotein classes. 50 µg of lipoprotein (VLDL, LDL, and HDL) proteins and lipoprotein-depleted plasma (LDP), isolated from healthy subjects were separated by SDS-PAGE, and subjected to immunoblotting analysis. Densitometric analysis of PCYOX1 protein content (% of total immunoreactive PCYOX1) in different lipoprotein classes (**p<0.05 vs.* VLDL). (B) Hepatic cells express PCYOX1 and secrete lipoprotein containing the enzyme. HepG2 cell lysate (50 µg) was separated by SDS-PAGE, and subjected to immunoblotting analysis. LDL were isolated from serum-free culture medium of 10⁸ HepG2 cells by ultracentrifugation and subjected to immunoblotting for the detection of PCYOX1. The image is representative of three independent experiments. Data published by Banfi C et al (Banfi, Brioschi et al. 2009).

Figure 2. Determination of H₂O₂ production by lipoprotein-associated PCYOX1 is able to produce H₂O₂. 50 µg of VLDL, LDL, and HDL proteins were incubated in the presence of PCYOX1 substrate, farnesylcysteine (FC) (200 µmol/L). H₂O₂ levels measured after 240 min were undetectable when lipoproteins were incubated with vehicle in the absence of FC. Values are the means ± SEM of 6 individual experiments (*p<0.05 vs VLDL). Data published by Banfi et al (Banfi, Brioschi et al. 2009).

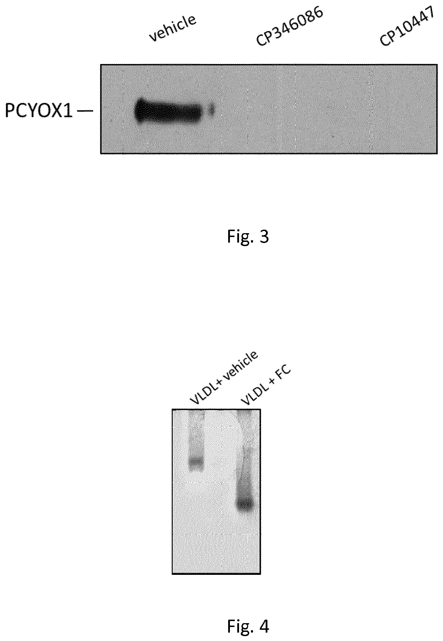

Figure 3. PCYOX1 is generated as part of the synthesis of nascent lipoprotein. HepG2 cells were treated with MTP inhibitors *(CP346086* 1µmol/L and *CP10447* 100 µmol/L, kindly provided by Dr Donnie W. Owens, Compound Transfer Manager at Pfizer, USA) or vehicle for 24 h. Lipoproteins (d<1.063) were isolated from the cell culture media by ultracentrifugation. Lipoprotein preparations were dialyzed extensively at 4°C against 5 mmol/L NH₄HCO₃ (pH 7.9), lyophilized, separated in SDS-PAGE, and transferred to nitrocellulose before Western blotting analysis for the detection of PCYOX1 .Representative image of 4 independent experiments.

Figure 4. H₂O₂ produced by lipoprotein-associated PCYOX1 oxidizes VLDL. 50 µg of VLDL were incubated with vehicle or PCYOX1 substrate farnesylcysteine (FC) (200 µmol/L) for 24 hours and then subjected to electrophoresis in agarose gel (0.7% in Tris 0.1 mol/L). Representative image of 4 independent experiments.

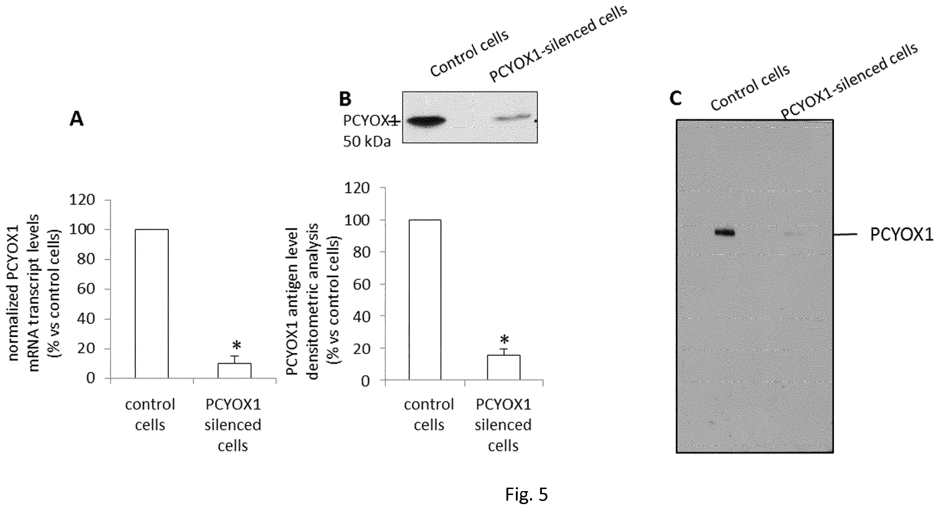

Figure 5. Stable PCYOX1 silencing significantly downregulates PCYOX1 mRNA and protein and abolishes the secretion of lipoprotein-associated PCYOX1 in HepG2 cells. Stable PCYOX1 silencing was obtained on HepG2 cells using PCYOX1 shRNA Plasmids as described in Methods. (A, B) PCYOX1 mRNA and protein levels were analyzed by qRT-PCR and immunoblotting, respectively. Values are the means ± SEM of 7 individual experiments *p<0.01 vs control cells. (C) Immunoblotting with anti- PCYOX1 antibody of lipoproteins isolated from cell culture media of control and PCYOX1-silenced HepG2 cells. Lipoproteins (d<1.063g/mL) were isolated from cell conditioned media by sequential ultracentrifugation. Lipoprotein preparations were dialyzed extensively at 4°C against 5 mmol/L NH₄HCO₃ (pH 7.9), lyophilized, separated in SDS-PAGE and transferred to nitrocellulose before Western blotting analysis for the detection of PCYOX1. Representative images of 7 independent experiments.

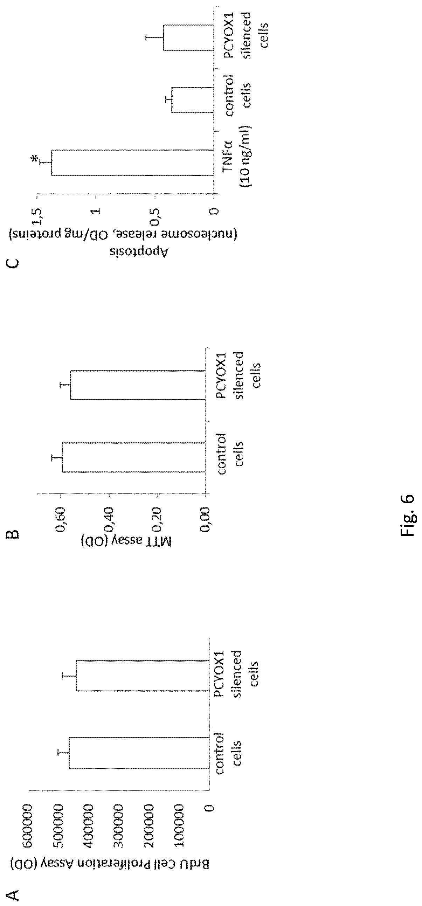

Figure 6. Stable PCYOX1 silencing did not affect cell growth and viability. (A) Effect of PCYOX1 silencing on cell proliferation. Cell proliferation was evaluated using the Cell proliferation ELISA, BrdU (Roche Diagnostic, Monza, Italy), in accordance with the manufacturer's instructions. Values are the means ± SEM of 4 individual experiments. (B) Effect of PCYOX1 silencing on cell viability. The MTT assay has been performed as described in the Methods section. Absorbance was recorded at 550 nm using the microplate spectrophotometer system. Values are the means ± SEM of 4 individual experiments. (C) Effect of PCYOX1 silencing on cell apoptosis. The histone-complexed DNA fragments (mono- and oligonucleosomes) were quantified using a one-step sandwich immunoassay (Roche Diagnostics, Mannheim, Germany), according to the manufacturer's instructions. Cells were incubated in serum free medium for 16 hours before the analysis. TNFα (10 ng/mL) was used as positive control. The data are expressed as absorbance/mg of cell proteins as determined by the Bradford protein assay. Values are the means ± SEM of 4 individual experiments. *p<0.01 vs control cells.

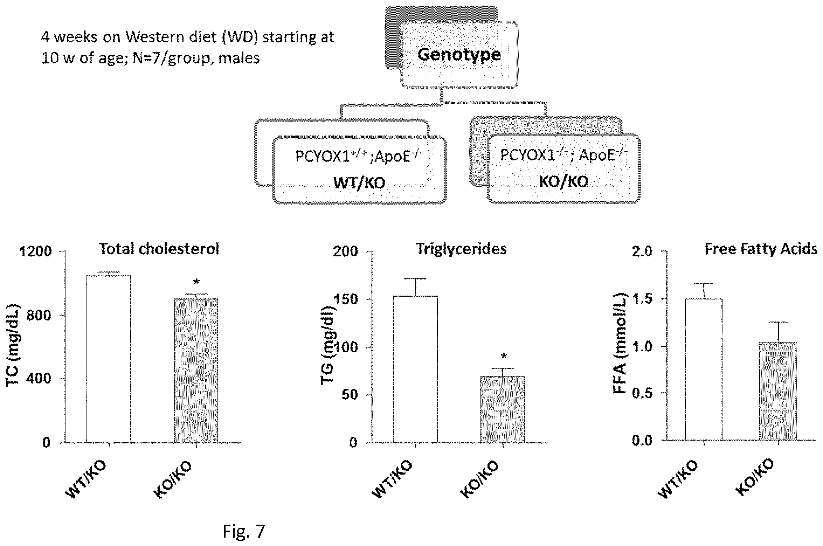

**Figure 7****. Effect of PCYOX1 deletion on plasma lipids in ApoE**^{-/-} **mice.** PCYOX1^{+/+}/apoE^{-/-} (WT/KO) and PCYOX1^{-/-}/apoE^{-/-} (KO/KO) male mice were fed a high-fat Western-type diet for 4 weeks. Blood samples were collected at sacrifice and plasma was isolated by centrifugation at 2500 rpm for 20 minutes. Concentrations of total cholesterol, triglycerides, and free fatty acids were determined using enzymatic colorimetric assays (Wako Chemicals and BioVision). Data are expressed as means ± SEM. Comparison between the 2 genotypes was performed by using the Student t test or Mann-Whitney U test as appropriate.

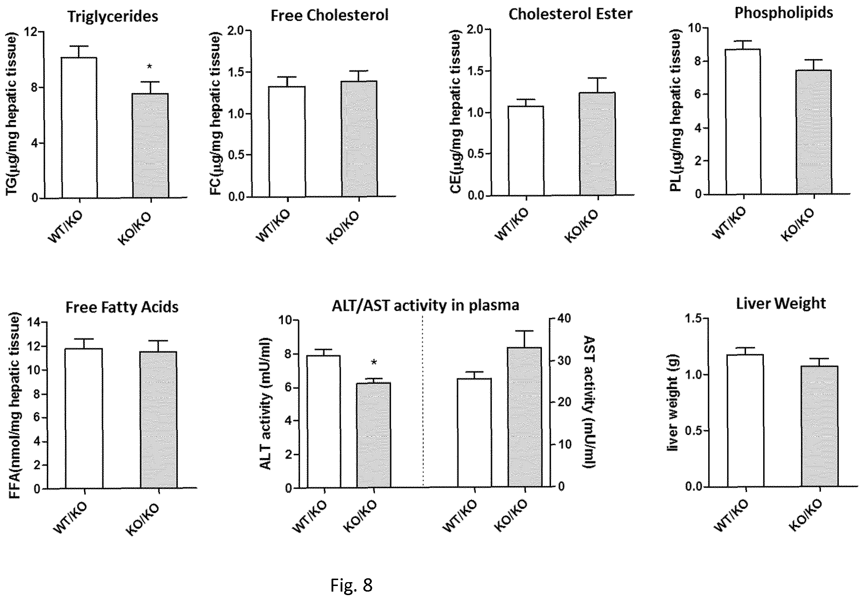

**Figure 8****. Effect of PCYOX1 deletion on liver function, weight, and lipid content in ApoE**^{-/-} **mice.** Liver tissue samples collected at sacrifice were homogenized and extracted in chloroform/methanol (2:1 v/v). Extracts were dried under N₂ flow and dissolved in isopropanol. Triglyceride, cholesterol, and phospholipid contents were measured using commercial kits (Wako Chemicals). Alanine aminotransferase (ALT) and aspartate aminotransferase (AST) activity in plasma were determined using enzymatic colorimetric assays (BioVision). Data are expressed as means ± SEM. Comparison between the 2 genotypes was performed by using the Student t test.

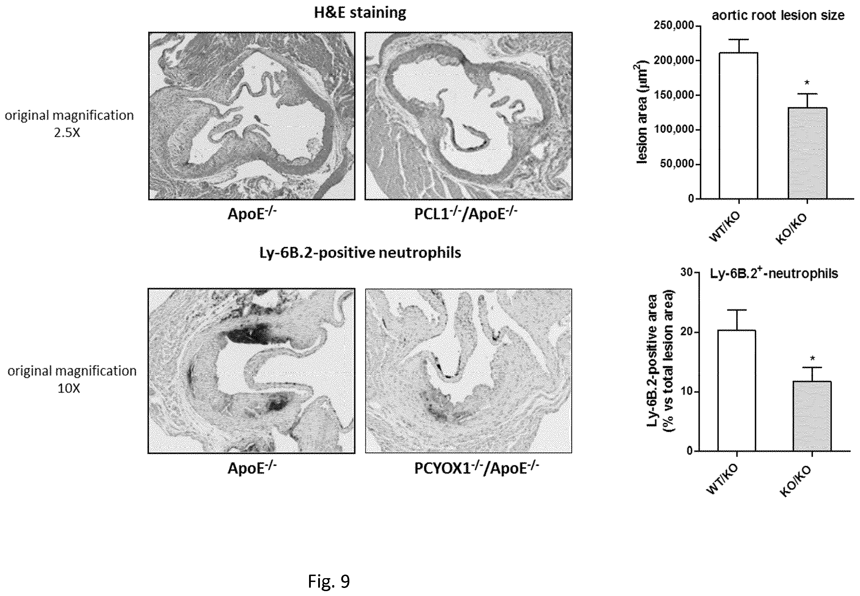

**Figure 9****. Effect of PCYOX1 deletion on the size and neutrophil content of early atherosclerotic lesions of ApoE**^{-/-} **mice.** At 4 weeks after initiation of the Western diet, mice were euthanized and the heart was harvested, immersion-fixed in 10% neutral buffered formalin and embedded in paraffin. Serial sections (7 µm in thickness) were cut through the aortic root. For each mouse, 5 sections separated by ≈70 µm were stained with hematoxylin-eosin to assess lesion size. To assess neutrophil infiltration, aortic root lesion sections were stained immunohistochemically for Ly-6B.2 and quantified by computer image analysis. Data are expressed as means ± SEM. Comparison between the 2 genotypes was performed by using the Student t test.

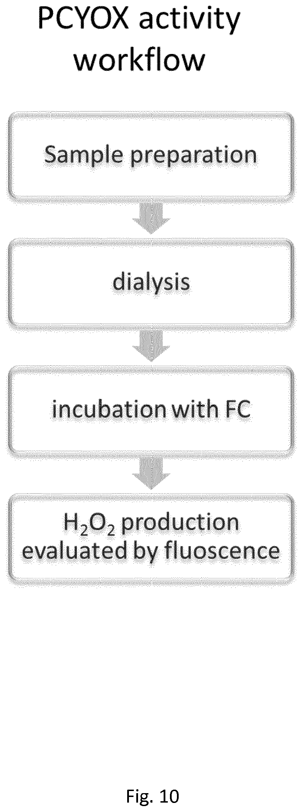

**Figure 10****. Workflow for the activity assay of PCYOX1**

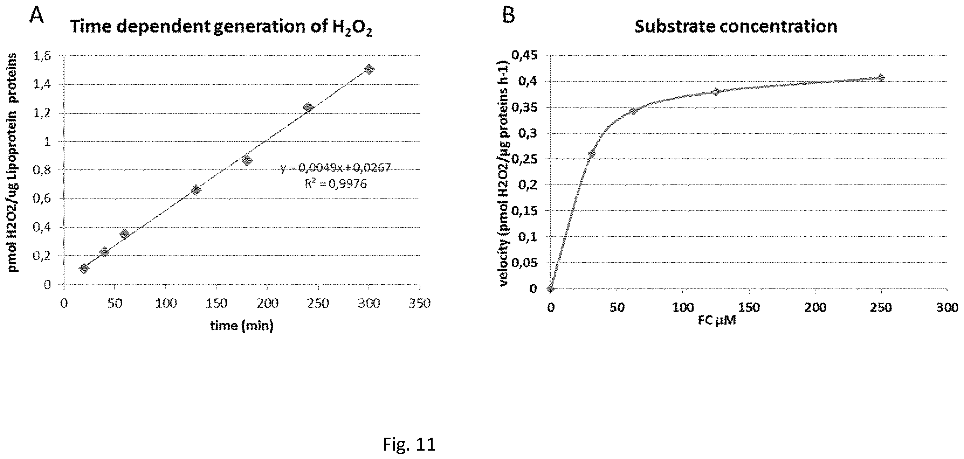

**Figure 11****. PCYOX1 activity assay.** A) Time dependent generation of H₂O₂ by PCYOX1-associated lipoprotein incubated with FC; B), velocity of the enzymatic reaction

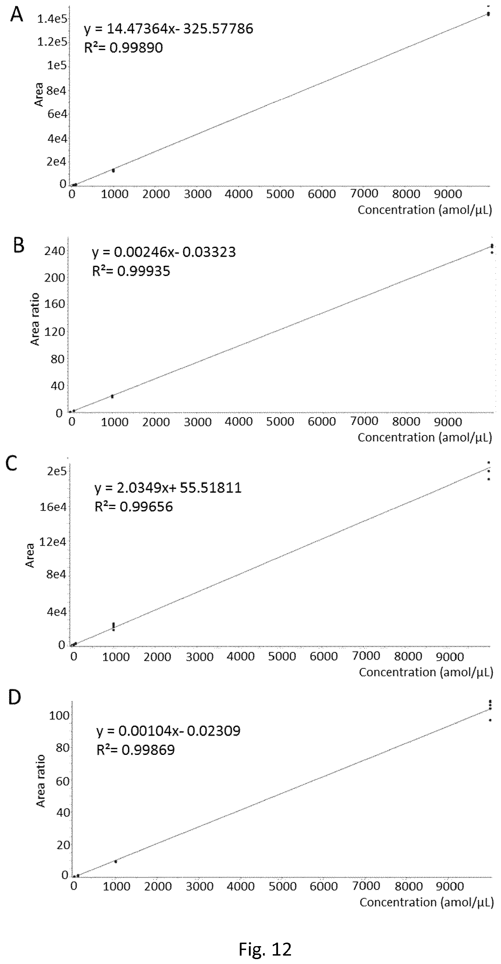

**Figure 12****. Standard curves of the two quantotypic human PCYOX1 peptides.** A) Standard curve of L11R 2y9 fragment (transition 588.33 →963.49) without labeled internal standard. B) Standard curve of L11R 2y9 fragment (transition 588.33 →963.49) with labeled internal standard. C) Standard curve of Y13K 3b7 fragment (transition 523.9->865.35) without labeled internal standard. D) Standard curve of Y13K 3b7 fragment (transition 523.9→865.35) with labeled internal standard. Equations and R² are reported for each curve.

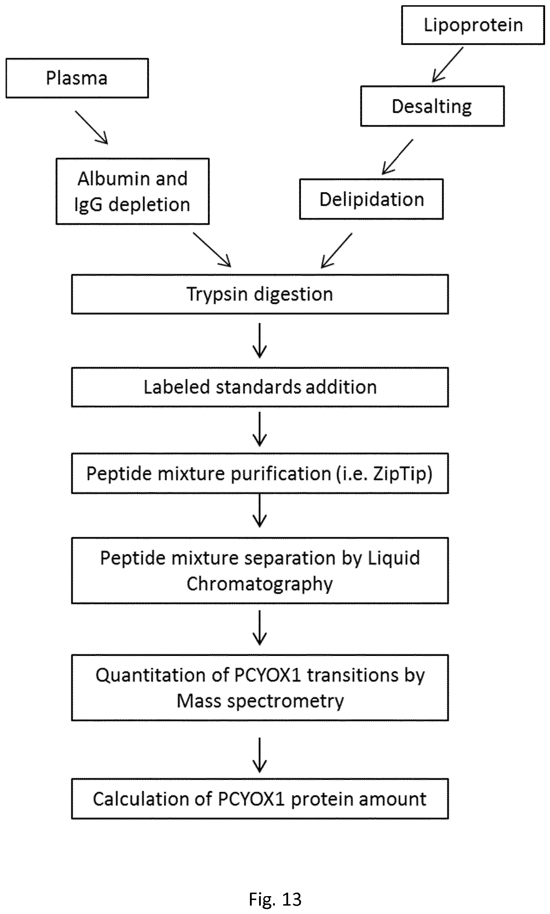

**Figure 13****. Workflow for MRM analysis of human PCYOX1 protein**

### EXAMPLES

### METHODS

**Lipoprotein isolation.** Blood from fasting healthy volunteers was collected in Na₂EDTA (1 mg/mL). Plasma was prepared by low speed centrifugation at 4°C, and lipoprotein isolation started within 1 h from blood collection. Lipoprotein were separated by density gradient ultracentrifugation (Havel, Eder et al. 1955): *d*,1.006 g/mL for very low-density lipoprotein (VLDL), d 1.019-1.050 g/mL for LDL, and 1.063-*d*,1.21 g/mL for HDL using a Beckman Ti 70.1 rotor. Lipoprotein classes may be isolated all together at d, 1.21-1.25 g/mL All lipoprotein preparations were dialyzed extensively at 4°C against 5 mmol/L NH₄HCO₃ (pH 7.9) containing 0.2 mmol/L EDTA. Protein concentrations were determined by a modified Lowry method (Lowry, Rosebrough et al. 1951). Electrophoretic mobility was assessed onto agarose gel (0.8% agarose in Tris 0.1 mol/L, pH 8.6) stained with Sudan Black.

**Silencing.** Stable PCYOX1 silencing was obtained on HepG2 cells using PCYOX1 shRNA Plasmids (Santa Cruz Biotecnologies), a pool of 3 target-specific lentiviral vector plasmids each encoding 19-25 nt (plus hairpin) shRNAs designed to knock-down PCYOX1 gene expression. An shRNA plasmid encoding for a scrambled shRNA sequence that does not lead to the specific degradation of any cellular message was used as negative control (control cells). Each plasmid contains a puromycin resistance gene for the selection of cells stably expressing shRNA. Transfection was performed on 50-70% confluent cells in antibiotic free normal growth medium supplemented with FBS. Cells were treated for 24 hours with 0.5 µg of plasmid and 2 µl of trasfection reagent (SantaCruz Biotechnologies) for each well of a 6 wells/plate and then grown in complete medium containing 1 µg/ml puromycin to enable selection.

**Cell proliferation assay.** Cell proliferation was evaluated using the Cell proliferation ELISA, BrdU (Roche Diagnostic, Monza, Italy), in accordance with the manufacturer's instructions. Briefly, the cells were grown in 96-well tissue-culture microplates and labelled by the addition of BrdU for 4 h. After removing the labelling medium, the cells were fixed, and the DNA was denatured in one step by adding FixDenat buffer. After removing the buffer, the anti-BrdU-POD antibody was added (which binds to the BrdU incorporated into the newly synthesised cellular DNA in place of thymidine). The immune complexes were detected by means of the subsequent substrate reaction. The reaction product was quantified by measuring light emission using a scanning multi-well luminometer (Mithras LB940, Berthold Technologies, Bad Wildbad, Germany).

**MTT assay.** The cells were incubated for 4 h at 37°C with 0.1 mg/mL of MTT, dissolved in serum-free medium. Washing with PBS was followed by the addition of DMSO (1 mL in a well of a six wells/plate) and gentle shaking for 5 min in order to ensure complete dissolution. Absorbance was recorded at 550 nm using the microplate spectrophotometer system.

**Apoptosis assay.** The histone-complexed DNA fragments (mono- and oligonucleosomes) were quantified using a one-step sandwich immunoassay (Roche Diagnostics, Mannheim, Germany), according to the manufacturer's instructions. Cells were incubated in serum free medium for 16 hours before the analysis. The data are expressed as absorbance/mg of cell proteins as determined by the Bradford protein assay.

**Mice and Diets.** PCYOX1^{-/-} mice (kindly provided by S.G. Young (Beigneux, Withycombe et al. 2002)) originally on a C57/BL6J-129SvJae mixed background were backcrossed into a C57/BL6J background and subsequently bred with apoE^{-/-} mice (Charles River Laboratories Italia). Intercrosses of the resulting apoE^{-/-}/PCYOX1^{+/-} mice yielded the offspring that entered the study. Genotyping for PCYOX1 and apoE was performed by PCR. Only male mice were used in these experiments to avoid the effects of hormonal changes on plasma lipids. Mice were housed in an air-conditioned room at 22°C±1°C with a 12-hour lighting schedule. The mice were fed a high-fat Western-type diet (0.15% cholesterol, 21% fat) beginning at 10 weeks of age and had free access to food and water. Four weeks after initiation of the dietetic regimens mice were anaesthetized by the intraperitoneal injection of a mixture of medetomidine hydrochloride (1mg/kg) and ketamine (75 mg/kg) and 0.5-1.0 mL of blood was collected into EDTA tubes by right ventricular puncture. The arterial tree was then perfused in situ with PBS via a cannula in the left-ventricular apex, and organs were harvested.

Experiments were conducted in accordance to the Guide for the Care and Use of Laboratory Animals published by the US National Institutes of Health (NIH Publication No. 85-23, revised 1996) with the approval of the responsible public authorities as required by the Italian Law in accordance with EU regulations (dl 4 March, 2014; European Directive 2010/63/UE).

### Plasma biochemical analyses

Plasma was isolated from blood collected at sacrifice by centrifugation at 2500 rpm for 20 minutes, divided into aliquotes and stored at -80°C. Concentrations of total cholesterol (TC), triglycerides (TG), and free fatty acids (FFA), and alanine aminotransferase (ALT) and aspartate aminotransferase (AST) activity in plasma were determined using enzymatic colorimetric assays (Wako Chemicals and BioVision) according to manufacturer's instructions.

### Hepatic lipid measurement

Liver tissues were homogenized using a Potter tissue grinder and extracted in chloroform/methanol (2:1 v/v) according to the classical Folch method. Extracts were dried under N₂ flow and dissolved in isopropanol. TG, total and free cholesterol, phospholipids, and FFA contents were measured using commercial kits (Biovision, USA) according to the manufacturer's instructions.

### Murine Aortic Root Lesion Analysis

The top half of the heart was immersed overnight in 10% neutral buffered formalin and embedded in paraffin. Serial sections (7 µm in thickness) were cut through a ≈350-µm segment of the aortic root, where all 3 valve leaflets were present. For each mouse, 5 sections separated by ≈70 µm were stained with hematoxylin-eosin. Images of the aortas were captured with Axioskop 2 plus microscope (Zeiss) and the atherosclerotic lesion area was quantified by computer image analysis, using Axiovision LE rel 4.8 software (Zeiss) by a blinded observer .

### Immunohistochemistry

To further characterize the morphological and cellular composition of atherosclerotic lesions, aortic root lesion sections were stained immunohistochemically for Ly-6B.2 to identify neutrophils. Endogenous peroxidase was quenched by incubation with hydrogen peroxide solution and nonspecific staining was blocked by using 10% normal goat serum. Sections were incubated first with primary monoclonal rat anti-mouse Ly-6B.2 antibody (1:200, AbD Serotec) for 1 hour at room temperature and then with a biotinylated goat anti-rat antibody (abcam, 1:200) for 30 minutes. Antigen-antibody complexes were visualized with a 30-minute exposure to Vectastain ABC Elite solution (Vector Laboratories), followed by 3,3'-diaminobenzidine (ImmPACT DAB, Vector Laboratories). For negative control the primary antibody was omitted and sections were incubated with normal goat serum. Stained sections were digitally captured, and immunohistochemistry was quantified with Axiovision image analysis software (Zeiss). Immunopositive area was determined by an independent observer that was blinded to genotype allocation, and expressed as percentage of the lesion area. The threshold for immunostaining was defined by sampling.

### PCOYX1 activity assay

All the reactions were performed using phosphate buffer 0.05 M pH7.4 as reaction buffer.

Amplex Red (Life technologies) was dissolved 10 mmol/L in DMSO. Horseradish Peroxidase (HRP, Life technologies) was dissolved in reaction buffer to obtained a stock solution of 10 U/mL. Amplex Red reaction solution was prepared in reaction buffer containing 0.2 U/mL HRP and 0.1 mmol/L immediately before use. 3% H₂O₂ was diluted in reaction buffer to generate a standard curve (19 nmol/L-10 µmol/L). FC was purchased from ARC (American Radiolabeled Chemicals, St Louis, MO, USA) as stock solution at 30 mmol/L in ethanol and was diluted in reaction buffer to working concentration ranging from 90-750 µmol/L. Ethanol was used as vehicle.

Lipoprotein pool samples were extensively dialysed against reaction buffer in glycerol-free Slide-A-Lyzer^{™} Dialysis Cassettes with a 3.5 KDa cut-off for 24 hours.

Standards (60 µL) or samples (40 µL containing 50 µg of proteins) were loaded in a 96 well black plate with clear bottom and 20 µL of FC or vehicle were added (final concentration 30-250 µmol/L) to the samples and incubated for 20 min-6 h at room temperature (25°C).

At the end of the incubation 60 uL of Amplex Red reaction solution were added and the fluorescence was read after 5 min on a microplate reader Infinite 200 (TECAN), equipped for excitation in the range of 530 ± 12.5 nm and fluorescence emission detection at 590 ± 17.5 nm.

For each point of the standard curve, the value derived from the no-H₂O₂ control was subtracted to create a linear regression curve. H₂O₂ production from PCYOX1 in a biological samples was calculated extrapolating from the standard curve the difference in fluorescence units between the samples treated with FC and the one incubated with ethanol (vehicle).

### Lipoprotein sample preparation for MRM assay

Lipoproteins pool obtained by ultracentrifugation at 1.21 g/mL density was desalted and delipidated with an optimized protocol using the Protein precipitation kit from Calbiochem. Briefly, 0,5 mg of lipoprotein were incubated for 60 min with the Precipitation Agent, provided in the kit, at -20°C. Proteins were pelleted by centrifugation at 3300 g for 10 min at room temperature and dissolved in 1 mL of Diethylether, incubated for 40 min on ice and centrifuged at 200g for 10min at 4°C. Proteins pellets were washed once with Diethylether, incubated and centrifuged as described above. Proteins pellets were then washed once a with Ethanol/Diethylether (3/1) with 40 min of incubation on ice. After centrifugation at 200g for 10min at 4°C, proteins pellets were washed once again with 1ml of Diethylether, incubated on ice for 40 min and centrifuged at 200g for 10min at 4°C. Alternative methods of delipidation may be used.

Finally, samples were dried under vacuum and dissolved in 25 mmol/L NH₄HCO₃ containing 0.1% RapiGest (Waters Corporation, Milford, MA, USA), sonicated and centrifuged at 13000g for 3 min. The protein concentrations determined using Bradford's method.

### Plasma samples preparation by immunopurification for MRM assay

To eliminate two of the most abundant plasma proteins, albumin and immunoglobulins, the plasma samples were pretreated using a ProteoPrep Blue Albumin Depletion Kit (Sigma, Milan, Italy) according to manufacturer's instruction before protein digestion. Briefly, 0.4 mLof ProteoPrep Blue Albumin depletion medium were equilibrated with a dedicated buffer, provided in the kit, 50 uL of plasma sample were incubated for 10 min at room temperature with the dry medium, centrifuged at 12000g for 60 seconds. Recovered eluated plasma was reapplied to the top of the medium bed and centrifuged as before for 60 seconds.

The protein concentrations were determined using Bradford's method.

### Protein digestion

Plasma proteins or lipoprotein proteins were diluted in 25 mmol/L NH₄HCO₃ containing 0.1% RapiGest (Waters Corporation, Milford, MA, USA), 200 µg of each sample were heated at 80 °C for 15 min, reduced with 5 mmol/L DTT at 60 °C for 15 min, and then carbamidomethylated with 10 mmol/L iodacetamide for 30 min at room temperature. Digestion was performed using 4 µg of sequencing grade trypsin (Promega, Milan, Italy) overnight at 37 °C. After digestion, 2% Trifluoracetic acid was added to hydrolyse RapiGest and inactivate trypsin, and the solution was incubated at 37 °C for 40 min before being vortexed and centrifuged at 13,000 g for 10 minutes.

### Trypsin Digest purification

Peptide purification was performed using ZipTip C18 (Millipore, Milan, Italy) in accordance with the manufacturer's instructions, and the peptides were then solubilised in 0.1 % v/v formic acid in water.

### Multiple Reaction Monitoring (MRM) Assay

Target peptides were subsequently quantified by MRM assay after the addition of heavy-labeled peptides at known concentrations, co-eluting in the chromatographic separation.

MRM assay was developed on QTRAP 4500 used as triple quadrupole mass spectrometer with electrospray ionization (ABSciex, Foster City, CA). Reverse phase separations were carried out with the Eksigent ekspert^{™} microLC 200 System on the ChromXP C18 column 500 µm × 100 mm, 3µM particle size (Eksigent, Redwood City, California).

Solvent A was LC-MS grade water with 0.1% (v/v) formic acid, and solvent B was LC-MS grade acetonitrile with 0.1% (v/v) formic acid. 5ul of samples (1.6 mg/ml total protein concentration) were loaded and eluted at a flow rate of 30 µL/min by increasing the solvent B concentration from 1% to 10% B over 10 min, and from 10 to 50% B, with a total run time of 17 min including 1.5 min wash at 95% B and 2.5 min equilibration. All of the analyses were made at least in triplicate. The mass spectrometer was operated in scheduled MRM determined with the Scheduled MRM^{™} algorithm (AB sciex, Foster City, CA). Q1 was set on the specific precursor m/z value and Q3 set to the specific m/z value corresponding to a specific fragment of a quantotypic peptide. Data were acquired in a positive ion mode with a 30msec dwell time using automatically determined tuning parameters and collision energies. Collision energy was identical for both labeled and unlabeled versions of each peptide.

For quantification, peak area of each peptide transition was determined using the MultiQuant software (ABSciex, Fosster city , CA). Unlabeled/labeled peak area ratios were calculated for each transition after careful verification of coelution profiles. Ratios obtained from the different transitions were used to calculate the corresponding average peptide ratio. Then, ratios obtained for the different quantotypic peptides were averaged to determine human PCYOX1 protein amount in plasma and lipoproteins.

### LC-MS^{E} analysis

Nanoscale LC separations of tryptic peptides for qualitative and quantitative multiplexed LC-MS^{E} analysis were performed using a nanoACQUITY system (Brioschi et al, 2013). Samples were injected into a 180 µm×2 cm Symmetry C18 (5 µm) trap column (Waters Corporation,Milford, MA, USA) for preconcentration and desalting. The peptide mixtures were subsequently directed from the pre-column to a 1.7 µm BEH 75 µm×250 mm analytical column (Waters Corporation, Milford, MA, USA). The samples were eluted at a flow rate of 300 nL/min by increasing the organic solvent concentration from 1 % to 40% B over 30 min, using 0.1% formic acid in water as reversed phase solvent A, and 0.1% formic acid in acetonitrile as reversed phase solvent B. The precursor ion masses and associated fragment ion spectra of the tryptic peptides were measured using a SYNAPT-MS, a hybrid quadruple orthogonal acceleration time-of-flight Q-Tof mass spectrometer (Waters Corporation, Milford, MA, USA) directly coupled to the chromatographic system. The time-of-flight analyser of the mass spectrometer was externally calibrated using [Glu1]-fibrinopeptide B (m/z 785.8426) from m/z 50 to 1990, and the data post-acquisition lock mass data was corrected using the monoisotopic mass of the doubly charged precursor of [Glu1]-fibrinopeptide B (m/z 785.8426) delivered to the mass spectrometer at 100 fmol/µL through a pressure pump. The reference sprayer was sampled every 30 s. Accurate mass data were collected in data independent acquisition mode by alternating low and high energy applied to the collision cell. The spectral acquisition time in eachmode was 0.6 s, with a 0.1 s inter-scan delay. In the low energy MS mode, data were collected at constant collision energy of 3 eV; in high energy mode, the collision energy was ramped from 12 to 35 eV during each 0.6 s scan. The RF applied to the quadruple mass analyser was adjusted in such a way that ions from m/z 300 to 2000 were efficiently transmitted, thus ensuring that any ion with amass of less than m/z 300 only arose from dissociations in the collision cell.

The data were processed and searched using ProteinLynx GlobalSERVER (PLGS) version 2.5.1 (Waters Corporation, Milford, MA, USA).. The proteins were identified by searching a human species-specific UniProt database (release 57.0; 20,333 entries). The following search criteria were used for protein identification: the default search parameters included the "automatic" setting for mass accuracy (approximately 10 ppm for precursor ions and 25 ppm for product ions); a minimum of one peptide match per protein, a minimum of three consecutive product ion matches per peptide, and a minimum of seven total product ion matches per protein; up to one missed cleavage site allowed; carbamidomethyl-cysteine as fixed modification; and methionine oxidation as variable modification.

### RESULTS

### PCYOX1 is generated as part of the synthesis of nascent lipoprotein.

Inhibition of MTP, a key enzyme for apolipoprotein secretion, abrogates PCYOX1 secretion suggesting that the association of PCYOX1 to lipoprotein follows the secretory pathways of apoB-containing lipoprotein in HepG2 cells (Fig. 3).

### H₂O₂ produced by lipoprotein-associated PCYOX1 oxidizes VLDL.

Incubation of VLDL with FC for 24 hours resulted in an increased electrophoretic mobility on agarose gel due to oxidation (Fig. 4). Therefore, PCYOX1 carried by human lipoproteins can itself generate an oxidant which in turn induces lipoprotein oxidation in vitro.

### Stable PCYOX1 silencing abolishes the secretion of lipoprotein-associated PCYOX1 in HepG2 cells and does not affect cell viability.

The shRNA used is a pool of 3 different shRNA plasmids:
sc-76093-SHA:
   Hairpin sequence:
      GATCCGGAAAGATGTGAAGATAGATTCAAGAGATCTATCTTCACATCTTTCCTTTTT (SEQ ID NO: 6)
   Corresponding siRNA sequences (sc-76093A):
      - Sense: GGAAAGAUGUGAAGAUAGAtt (SEQ ID NO: 7)
      - Antisense: UCUAUCUUCACAUCUUUCCtt (SEQ ID NO: 8)
   sc-76093-SHB:
      Hairpin sequence:
         GATCCCCACTCCGTTGAATCGAAATTCAAGAGATTTCGATTCAACGGAGTGGTTTTT (SEQ ID NO: 9)
      Corresponding siRNA sequences (sc-76093B):
         - Sense: CCACUCCGUUGAAUCGAAAtt (SEQ ID NO: 10)
         - Antisense: UUUCGAUUCAACGGAGUGGtt (SEQ ID NO: 11)
sc-76093-SHC:
   Hairpin sequence:
      GATCCGAAGCCCAATCTGTATCAATTCAAGAGATTGATACAGATTGGGCTTCTTTTT (SEQ ID NO: 12)
   Corresponding siRNA sequences (sc-76093C):
      - Sense: GAAGCCCAAUCUGUAUCAAtt (SEQ ID NO: 13)
      - Antisense: UUGAUACAGAUUGGGCUUCtt (SEQ ID NO: 14)

All sequences are provided in 5' ---> 3' orientation.

Stable PCYOX1 silencing was obtained on HepG2 cells (HepG2 cell line human, cod. 85011430, Sigma Aldrich) using PCYOX1 shRNA Plasmids (Santa Cruz Biotecnologies), a pool of 3 target-specific lentiviral vector plasmids each encoding 19-25 nt (plus hairpin) shRNAs designed to knock-down PCYOX1 gene expression (PCYOX1 shRNA Plasmid Cod. sc-76093-SH, Santa Cruz Biotecnologies). An shRNA plasmid encoding for a scrambled shRNA sequence that does not lead to the specific degradation of any cellular message was used as negative control (control cells). Each plasmid contains a puromycin resistance gene for the selection of cells stably expressing shRNA. PCYOX1 mRNA and protein levels were significantly downregulated in stable silenced cells (Fig.5A, B). Lipoproteins isolated from control and silenced cells do not contain PCYOX1 (Fig. 5C). Furthermore, shRNA delivery reagents allows efficient PCYOX1 gene silencing without affecting cellular viability and cell growth as assessed by the BrdU proliferation assay, MTT assay and apoptosis assay (Fig.6A, B, C).

### PCYOX1 knockdown reduces size and neutrophil content of early atherosclerotic lesions of ApoE^{-/-} mice.

To explore the potential role of PCYOX1 in lipid metabolism and atherosclerosis, the authors generated PCYOX1^{-/-}/apoE^{-/-} (double KO) mice and fed them with a western-type diet for 4 weeks (Fig. 7). Analysis of plasma lipid profile showed that at this time point double KO mice had significantly lower levels of TC (Total Cholesterol) and TG (Triglycerides) compared to apoE^{-/-} control mice (Fig. 7), which suggests that PCYOX1 has a role in lipid metabolism in this animal model. Plasma levels of FFA (Free Fatty Acid) were also lower in double KO mice, although this difference did not reach statistical significance (Fig. 7). This effect was accompanied by a decrease in hepatic triglyceride content (Fig. 8), which support the hypothesis that PCYOX1 is involved in lipid metabolism. No signs of hepatic dysfunction (as assessed by measuring plasma ALT and AST activity) or alteration in liver weight was detected in double KO mice compared to apoE^{-/-} controls (Fig. 8). Analysis of aortic root sections revealed that PCYOX1 deletion resulted in a reduction of lesion size and neutrophil content at the time point examined, which suggest that PCYOX1 might promote early atherosclerosis in apoE^{-/-} mice (Fig. 9).

### Development of an assay to measure total activity of plasmatic PCYOX, and to measure the alteration of the activity of PYCOX1

In the present invention inventors developed an accurate workflow and assay for assessing PCYOX1 activity in human biological samples avoiding the use of radioactive compounds. The assay of the invention is applicable in principle to the plasma or serum of any mammal, but is, of course, mainly of interest in relation to humans. One purpose of the assay is to detect abnormalities of PCYOX1 in human diseases.

The assay is based on the measurement of H₂O₂ production by PCYOX1 in the presence of its substrate, FC. The workflow has been optimized for the analysis of total plasmatic PCYOX1 activity. Lipoproteins fraction (density 1.21-1.25 g/mL) were isolated as described in the Methods section, and desalted in phosphate buffer 0.05 mol/L, pH 7.4 for 24 hours by means of membrane dialysis. Lipoprotein fraction was incubated for a 2-4 hours with FC or vehicle as control. At the end of incubation Amplex Red reagent and HRP were added to measure the amount of H₂O₂ generated as unit of fluorescence monitored at λex = 530-560 nm, λem = 590 nm. Results were calculated as delta between FC-incubated sample and vehicle-incubated sample and concentration were extrapolated from the standard curve obtained with known concentrations of H₂O₂. A schematic workflow is shown in Figure 10.

Experiments were performed to select the incubation time, the FC substrate concentration and the amount of lipoprotein fraction used.

Figure 11A shows the time dependent generation of H₂O₂ by PCYOX1-associated lipoprotein incubated with FC, which produces a linear enzymatic response (R² = 0.997) for the complete duration of the assay from 20 minutes to six hours. Two hours of incubation were selected for subsequent analysis on the basis that it is better to perform an enzymatic assay under initial velocity condition where less than 10% of the substrate is used, only if the measurement could be considered accurate in relation to the standard curve. Further implementation of the invention may include longer incubation time for the detection of low activity levels of PCYOX1 .

Figure 11B shows the velocity of the enzymatic reaction in relation to FC concentration. The reaction velocity was determined by plotting the H₂O₂ formation obtained with different substrate concentrations (from 30 µmol/L to 250 µmol/L FC) over time, with the slope of each line representing the velocity. The velocity was then plotted against substrate concentration in a non linear regression analysis, that allowed to determine that 125 µmol/L FC represents the optimal concentration.

The developed assay presents the following characteristics of reproducibility: Intra assay precision: CV 2.68%; Inter-assay precision. CV 12.4%.

Furthermore, enzymatic activity is preserved even if the biological sample is subjected to one freeze-thawing cycle (recovery 101.1 ± 1.83%)

The specificity of the assay is inferred by the fact the lipoprotein isolated from PCYOX1-silenced cells do not generate H₂O₂ in comparison with lipoprotein derived from wild type cells. In addition lipoprotein isolated from healthy subject carrying different amount of PCYOX1 generate H₂O₂ proportionally to the amount of PCYOX1 (R² = 0.892).

In other implementation of the assay, isolated lipoprotein classes (VLDL, LDL, HDL) from human and non human species may be used.

### Identification of optimal fragments peptides from the human PCYOX1 protein for MRM assay

The human PCYOX1 recombinant protein was treated using trypsin to digest the protein. All resulting protein fragments were analyzed by tandem mass spectrometry on a SYNAPT-MS, a hybrid quadruple orthogonal acceleration time-of-flight Q-Tof mass spectrometer (Waters Corporation, Milford, MA, USA) directly coupled to a nanoAquity UPCL system for chromatographic separation, and fragment peptides from the human PCYOX1 protein were identified.

All peptides generated by a trypsin digestion method from the full length human PCYOX1 protein can be potentially measured, but preferred peptides are those that are proteotypic for human PCYOX1 ( unique to the protein of interest) and with the following features: highly detectable by LC-MS, peptide length of 7-25 amino acids, no peptides with missed cleavages sites, no peptides with residues susceptible to artifactual modifications. Peptides with these characteristics can be considered quantotypic.

All the information obtained from the digestion of the recombinant protein were further compared with the data reported in the public repository "Peptide Atlas" containing all the data about the detection of human PCYOX1 peptides in different biological samples analyzed by different laboratories with different instrumentations.

Selected quantotypic peptides for human PCYOX1 are reported in the Table 1

**Table 1. List of the quantotypic peptides selected for MRM assay of human PCYOX1**

| **NName** | **Sequence** | **position** |
|---|---|---|
| Y-13-K | YQSHDYAFSSVEK | 169-181 OF SEQ ID NO: 3 (SEQ ID NO: 4) |
| L-11-R | LV**C***SGLLQASK | 256-266 OF SEQ ID NO: 3 (SEQ ID NO: 5) |

| | | |
|---|---|---|
| * Cysteine was carbamidomethylated | | |

### Development of MRM Assay for the quantitation of human PCYOX1 protein amount

Suitable transition ions for the two PCYOX1 quantotypic peptides were selected to monitor a total of six fragment peptides (Table 2) based on the highest signal to noise ratio and/or the lowest standard deviation between replicate analyses obtained after MS tuning.

To facilitate accurate quantitation of the surrogate peptides by mass spectrometry, a set of isotopically-labeled synthetic versions of the surrogate peptides may be added in known amounts to the sample for use as internal standards. Since the isotopically-labeled peptides have physical and chemical properties substantially identical to the corresponding surrogate peptide, they co-elute from the chromatographic column and are easily identified on the resultant mass spectrum. In the method used in this invention, labeled peptides were added to the complex biological mixtures after trypsin digestion. In other implementations of the invention, labeled peptides may be added to the sample prior to the purification and/or digestion steps.

The following sets of transitions (Table 2) have been found to provide high degrees of sensitivity and selectivity for detection/quantitation of the associated surrogate peptides. Alternative implementations of the assay may use a greater or lesser number of transitions for each surrogate peptide; however, it is generally advisable to use at least two transitions for each peptide to ensure an acceptable degree of specificity and avoid misidentification of targeted substances.

**Table 2. List of the selected peptides and their corresponding transitions**

| **Name** | **Sequence** | **m+h** | **Precursor masses** | **Products masses** | **Fragment** |
|---|---|---|---|---|---|
| Y-13-K light | YQSHDYAFSSVEK (SEQ ID NO: 4) | 1560.70 | 520.90 (charge 3+) | 865.35 | b7 |
| | | | | 696.36 | y6 |
| | | | | 549.29 | y5 |
| L-11-R light | LV**C***SGLLQASK (SEQ ID NO: 5) | 1175.64 | 588.33 (charge 2+) | 963.49 | y9 |
| | | | | 803.46 | y8 |
| | | | | 716.43 | y7 |
| Y-13-K heavy | YQSHDYAFSSVE[K(13C6; 15N2)] (SEQ ID NO: 15) | 1568.70 | 523.9 (charge 3+) | 865.35 | b7 |
| | | | | 704.36 | y6 |
| | | | | 557.29 | y5 |
| L-11-R heavy | LV**C***SGLLQAS[K(13C6; 15N2)] (SEQ ID NO: 16) | 1183.64 | 592.33 (charge 2+) | 971.49 | y9 |
| | | | | 811.46 | y8 |
| | | | | 724.43 | y7 |

| | | | | | |
|---|---|---|---|---|---|
| *Cysteine was carbamidomethylated | | | | | |

The samples containing the surrogate peptides (and associated internal standards) were chromatographically separated by using the HPLC column and elution protocol described in Methods in order to temporally isolate different compounds in the prepared sample and simplify the process of identifying and quantifying substances in the mass spectra. In other implementations of the invention other HPLC configurations and methods may be used. In a preferred mode of the invention, chromatographic separation is performed on-line, such that the column eluate is directly introduced into the inlet of the ion source of the mass spectrometer, for example through an electrospray ionization probe. Alternatively, fractions of the eluate may be collected and stored for later mass analysis.

MS data were acquired on the triple quadrupole mass spectrometer operated in scheduled MRM after automatic optimization of the tuning parameters and collision energies for each peptide. Other implementation of this invention could include different triple quadrupole mass spectrometers or acquisition mode.

Once the mass spectrometric analysis has been completed, the quantities of the surrogate peptides in the sample may be determined by integration of the relevant peak areas, as known in the prior art. Unlabeled/labeled peak area ratios were calculated for each transition after careful verification of coelution profiles. Ratios obtained from the different transitions were used to calculate the corresponding average peptide ratio. Then, ratios obtained for the different quantotypic peptides were averaged to calculate the protein ratio and determine human PCYOX1 protein amount.

Quantitation could be achieved either using the best transition as quantifier and the other as qualifier (to confirm protein identity) or using all the transitions as quantifiers and calculating the amount as mean of the two/four results. According to this invention we mediated the results obtained with 3 transitions for each peptide. In other implementations of the invention the other choice may be followed depending on the sample complexity.

To test the linearity of the method and the limit of quantitation (LOQ), light peptides were diluted with 0.1% Formic Acid (FA) to give the following concentrations (10, 50, 100 amol/µL and 1, 10, 100 fmol/µL). Dilution series were prepared with and without the corresponding labeled peptides (conc. 10 fmol/µL). 5 µL of each sample were injected and were measured five times.

MRM transitions exhibited linear responses (Figure 12) relative to human PCYOX1 peptides concentration, with a LOQ of 50 amol/µL for L11R and 100 amol/µL for Y13K in water. The coefficient of variation for quintuplicate measurements ranged from 3-16% for all peptides with less than <5% chromatographic drift between replicates, indicating high accuracy and reproducibility between measurements.

In order to apply the above described MRM assay to complex biological matrices we developed the following workflow that was applied both to plasma and lipoprotein samples (Figure 13).

Plasma sample was treated with ProteoPrep Albumin depletion kit, as described in Methods, to eliminate the two most abundant plasma proteins (albumin and Immunoglobulins). In other implementation of the assay, plasma samples may be analyzed without the purification from plasma proteins (e.g. albumin and Immunoglobulins). After tryptic digestion the sample was spiked with heavy peptides in a concentration of 10 fmol/µL. 5 µL plasma digest (total proteins concentration of 1.6 mg/mL) was measured in scheduled MRM in quadruplicate. The MultiQuant^{™} software was used to calculate the MRM ratio. The average for all transitions from both peptides and four runs was 4.5 ± 0.2 fmol/µL native peptides in plasma. The CVs for all peptides from four runs were below 7.5%.

Lipoprotein sample was desalted with the Protein Precipitation kit and delipidated as described in Methods. Lipoprotein digest was spiked with heavy peptides in a concentration of 100 fmol/µL. 5 µL sample (total proteins concentration of 1.6 mg/mL) was measured in scheduled MRM in quadruplicate. The MultiQuant^{™} software was used to calculate the MRM ratio.

The average for all transitions from both peptides in four runs was 21 ± 1.04 fmol/µL native peptides in plasma. The CVs for all peptides from four runs were below 2.5%

In conclusion, MRM mass spectrometry technique practiced in accordance with the present invention enables the monitoring of human PCYOX1 in different biological samples, via detection and quantitation of the surrogate peptides and their transitions according to the workflow for sample preparation and data acquisition.

### REFERENCES

Banfi, C., M. Brioschi, et al. (2009). "Proteomic analysis of human low-density lipoprotein reveals the presence of prenylcysteine lyase, a hydrogen peroxide-generating enzyme." Proteomics 9(5): 1344-1352.
Beigneux, A., S. K. Withycombe, et al. (2002). "Prenylcysteine lyase deficiency in mice results in the accumulation of farnesylcysteine and geranylgeranylcysteine in brain and liver." J Biol Chem 277(41): 38358-38363.
Bhatt, D. L., J. S. Hulot, et al. (2014). "Antiplatelet and anticoagulation therapy for acute coronary syndromes." Circ Res 114(12): 1929-1943.
Digits, J. A., H. J. Pyun, et al. (2002). "Stereospecificity and kinetic mechanism of human prenylcysteine lyase, an unusual thioether oxidase." J Biol Chem 277(43): 41086-41093.
Do, R. Q., S. J. Nicholls, et al. (2014). "Evolving targets for lipid-modifying therapy." EMBO Mol Med 6(10): 1215-1230.
Havel, R. J., H. A. Eder, et al. (1955). "The distribution and chemical composition of ultracentrifugally separated lipoproteins in human serum." J Clin Invest 34(9): 1345-1353.
Ku SH, et al. (2015). Chemical and structural modifications of RNAi therapeutics. Adv Drug Deliv Rev. pii: S0169-409X(15)00240-9. doi: 10.1016/j.addr.2015.10.015
Lowry, O. H., N. J. Rosebrough, et al. (1951). "Protein measurement with the Folin phenol reagent." J Biol Chem 193(1): 265-275.
Lu, J. Y. and S. L. Hofmann (2006). "Thematic review series: lipid posttranslational modifications. Lysosomal metabolism of lipid-modified proteins." J Lipid Res 47(7): 1352-1357.
Meir, K. S. and E. Leitersdorf (2004). "Atherosclerosis in the apolipoprotein-E-deficient mouse: a decade of progress." Arterioscler Thromb Vase Biol 24(6): 1006-1014.
Mohr SE,et al. (2014) RNAi screening comes of age: improved techniques and complementary approaches. Nat Rev Mol Cell Biol. 15(9):591-600. doi: 10.1038/nrm3860
Reuter, S., S. C. Gupta, et al. (2010). "Oxidative stress, inflammation, and cancer: how are they linked?" Free Radic Biol Med 49(11): 1603-1616.
Tschantz, W. R., J. A. Digits, et al. (2001). "Lysosomal prenylcysteine lyase is a FAD-dependent thioether oxidase." J Biol Chem 276(4): 2321-2324.
Wojtkowiak, J. W., R. A. Gibbs, et al. (2009). "Working together: Farnesyl transferase inhibitors and statins block protein prenylation." Mol Cell Pharmacol 1(1): 1-6.
Young, S. G., S. H. Yang, et al. (2013). "Targeting protein prenylation in progeria." Sci Transl Med 5(171): 171ps173.
Zhang, F. L. and P. J. Casey (1996). "Protein prenylation: molecular mechanisms and functional consequences." Annu Rev Biochem 65: 241-269.
Zhang L, (1997)Isolation and characterization of a prenylcysteine lyase from bovine brain. J. Biol Chem. ;272(37):23354-9.
Zhou, M., Z. Diwu, et al. (1997). "A stable nonfluorescent derivative of resorufin for the fluorometric determination of trace hydrogen peroxide: applications in detecting the activity of phagocyte NADPH oxidase and other oxidases." Anal Biochem 253(2): 162-168.

## Claims

1. A molecule able to inhibit or block prenylcysteine oxidase I (PCYOX1) expression and/or function selected from the group consisting of:
a) polynucleotide able to specifically inhibit the expression of the gene PCYOX1 selected from a siRNA, a shRNA, a microRNA or an antisense oligonucleotide;
b) a vector comprising or expressing said polynucleotide of a);
c) farnesal or farnesol
for use in the treatment and/or prevention of oxidative stress-related degenerative diseases selected from the group consisting of: atherothrombosis, atherosclerosis, diabetes, cardiovascular diseases, chronic inflammation, stroke and septic shock, cancer, aging, preferably of atherothrombosis.

2. The molecule for use according to claim 1 being farnesal and farnesol.

3. The molecule for use according to claim 1 wherein the shRNA comprises the SEQ ID NO: 6, SEQ ID NO: 9 or SEQ ID NO: 12, or a functional derivative thereof and/or the siRNA comprises at least one of the sequences selected from the group consisting of: SEQ ID NO: 7, SEQ ID NO: 8, SEQ ID NO: 10, SEQ ID NO:11, SEQ ID NO: 13 and SEQ ID NO: 14, or a functional derivative thereof, preferably wherein the molecule is a shRNA consisting of the SEQ ID NO: 6, SEQ ID NO: 9 or SEQ ID NO: 12, or a functional derivative thereof or is a siRNA consisting of the SEQ ID NO: 7, SEQ ID NO: 8, SEQ ID NO: 10, SEQ ID NO:11, SEQ ID NO: 13 or SEQ ID NO: 14, or a functional derivative thereof,
wherein the functional derivative inhibits the expression of the PCYOX1 gene.

4. A composition comprising the molecule for use as defined in any one claims 1-3, combined with at least one anti-atherothrombotic agent, i.e. agents able to reduce low density lipoprotein, lipoprotein(a), triglyceride-rich lipoprotein and/or at least one agent able to modulate high density lipoprotein levels and function and/or at least one anticoagulants and antiplatelet drugs for use in the treatment and/or prevention of oxidative stress-related degenerative diseases selected from the group consisting of: atherothrombosis, atherosclerosis, diabetes, cardiovascular diseases, chronic inflammation, stroke and septic shock, cancer, aging, preferably of atherothrombosis.

5. A pharmaceutical composition comprising the molecule for use as defined in any one of 1-3 or the composition for use according to claim 4 and at least one pharmaceutically acceptable carrier, for use in the treatment and/or prevention of oxidative stress-related degenerative diseases selected from the group consisting of: atherothrombosis, atherosclerosis, diabetes, cardiovascular diseases, chronic inflammation, stroke and septic shock, cancer, aging, preferably of atherothrombosis.

6. A genetically engineered host cell transduced, transformed or transfected with and able to express in suitable conditions the shRNA or the siRNA or a functional derivative thereof as defined in claim 3 for use in the treatment and/or prevention of oxidative stress-related degenerative diseases, preferably of atherothrombosis.

## Patentansprüche

1. Molekül, das in der Lage ist, die Expression und/oder die Funktion von Prenylcysteinoxidase I (PCYOX1) zu hemmen oder zu blockieren, ausgewählt aus der Gruppe bestehend aus:
a) einem Polynukleotid, das in der Lage ist, die Expression des PCYOX1-Gens spezifisch zu hemmen, ausgewählt aus einer siRNA, einer shRNA, einer MikroRNA oder einem Antisense-Oligonukleotid;
b) einem Vektor umfassend oder exprimierend das Polynukleotid von a);
c) Farnesal oder Farnesol,
zur Verwendung bei der Behandlung und/oder Vorbeugung von mit oxidativem Stress verbundenen degenerativen Erkrankungen, ausgewählt aus der Gruppe bestehend aus Atherothrombose, Atherosklerose, Diabetes, kardiovaskulären Erkrankungen, chronischer Entzündung, Schlaganfall und septischem Schock, Krebs, Altern, vorzugsweise von Atherothrombose.

2. Molekül zur Verwendung nach Anspruch 1, bei dem es sich um Farnesal und Farnesol handelt.

3. Molekül zur Verwendung nach Anspruch 1, wobei die shRNA die SEQ ID NR.: 6, SEQ ID NR.: 9 oder SEQ ID NR.: 12 oder ein funktionelles Derivat davon umfasst und/oder die siRNA mindestens eine der Sequenzen ausgewählt aus der Gruppe bestehend aus: SEQ ID NR.: 7, SEQ ID NR.: 8, SEQ ID NR.: 10, SEQ ID NR.: 11, SEQ ID NR.: 13 und SEQ ID NR.: 14 oder ein funktionelles Derivat davon umfasst, wobei es sich bei dem Molekül vorzugsweise um eine shRNA bestehend aus der SEQ ID NR.: 6, SEQ ID NR.: 9 oder SEQ ID NR.: 12 oder ein funktionelles Derivat davon handelt oder es sich dabei um eine siRNA bestehend aus der SEQ ID NR.: 7, SEQ ID NR.: 8, SEQ ID NR.: 10, SEQ ID NR.: 11, SEQ ID NR.: 13 oder SEQ ID NR.: 14 oder ein funktionelles Derivat davon handelt,
wobei das funktionelle Derivat die Expression des PCYOX1-Gens hemmt.

4. Zusammensetzung umfassend das Molekül zur Verwendung nach der Definition in einem der Ansprüche 1 bis 3, kombiniert mit mindestens einem antiatherothrombotischen Mittel, d. h. Mitteln, die in der Lage sind, Lipoprotein niedriger Dichte, Lipoprotein(a), triglyceridreiches Lipoprotein zu reduzieren, und/oder mindestens einem Mittel, das in der Lage ist, Spiegel und Funktion von Lipoprotein hoher Dichte zu modulieren, und/oder mindestens einem Antikoagulans und Thrombozytenaggregationshemmer zur Verwendung bei der Behandlung und/oder Vorbeugung von mit oxidativem Stress verbundenen degenerativen Erkrankungen, ausgewählt aus der Gruppe bestehend aus Atherothrombose, Atherosklerose, Diabetes, kardiovaskulären Erkrankungen, chronischer Entzündung, Schlaganfall und septischem Schock, Krebs, Altern, vorzugsweise von Atherothrombose.

5. Pharmazeutische Zusammensetzung umfassend das Molekül zur Verwendung nach der Definition in einem der Ansprüche 1 bis 3 oder die Zusammensetzung zur Verwendung nach Anspruch 4 und mindestens einen pharmazeutisch akzeptablen Träger zur Verwendung bei der Behandlung und/oder Vorbeugung von mit oxidativem Stress verbundenen degenerativen Erkrankungen, ausgewählt aus der Gruppe bestehend aus Atherothrombose, Atherosklerose, Diabetes, kardiovaskulären Erkrankungen, chronischer Entzündung, Schlaganfall und septischem Schock, Krebs, Altern, vorzugsweise von Atherothrombose.

6. Genetisch veränderte Wirtszelle, die mit der shRNA oder der siRNA oder einem funktionellen Derivat davon nach der Definition in Anspruch 3 transduziert, transformiert oder transfiziert ist und zu deren Expression in der Lage ist, zur Verwendung bei der Behandlung und/oder Vorbeugung von mit oxidativem Stress verbundenen degenerativen Erkrankungen, vorzugsweise von Atherothrombose.

## Revendications

1. Molécule capable d'inhiber ou de bloquer l'expression et/ou la fonction de la prénylcystéine oxydase I (PCYOX1) choisie dans le groupe constitué par :
a) un polynucléotide capable d'inhiber spécifiquement l'expression du gène PCYOX1 choisi parmi un ARNsi, un ARNsh, un microARN ou un oligonucléotide antisens ;
b) un vecteur comprenant ou exprimant ledit polynucléootide de a) ;
c) le farnésal ou le farénsol
destiné à être utilisé dans le traitement et/ou la prévention de maladies dégénératives associées aux stress oxydatif choisies dans le groupe constitué par : l'athérothrombose, l'athérosclérose, le diabète, les maladies cardiovasculaires, l'inflammation chronique, l'accident vasculaire cérébral et le choc septique, le cancer, le vieillissement, de préférence de l'athérothrombose.

2. Molécule destinée à être utilisée selon la revendication 1 étant le farnésal et le farnésol.

3. Molécule destinée à être utilisée selon la revendication 1 dans laquelle l'ARNsh comprend les SEQ ID n° 6, SEQ ID n° 9 oU SEQ ID n° 12 ou un de leurs dérivés fonctionnels et/ou l'ARNsi comprend au moins une des séquences choisies dans le groupe constitué par : SEQ ID N° 7, SEQ ID n° 8, SEQ ID n° 10, SEQ ID n° 11, SEQ ID n° 13 et SEQ ID n° 14 ou un de leurs dérivés fonctionnels, de préférence la molécule étant un ARNsh constitué de SEQ ID n° 6, SEQ ID n° 9 ou SEQ ID n° 12 ou un de leurs dérivés fonctionnels ou est un ARNsi constitué de SEQ ID n° 7, SEQ ID n° 8, SEQ ID n° 10, SEQ ID n° 11, SEQ ID n° 13 ou SEQ ID n° 14 ou un de leurs dérivés fonctionnels,
le dérivé fonctionnel inhibant l'expression du gène PCYOX1.

4. Composition comprenant la molécule destinée à être utilisée comme défini dans l'une quelconque des revendications 1 à 3, combinée avec au moins un agent anti-athérothrombotique, c'est-à-dire les agents capables de réduire la lipoprotéine de faible densité, la lipoprotéine (a), la lipoprotéine riche en triglycérides et/ou au moins un agent capable de moduler les niveaux et la fonction de la lipoprotéine de haute densité et/ou au moins un anticoagulant et médicament antiplaquettaire destinés à être utilisés dans le traitement et/ou la prévention de maladies dégénératives associées au stress oxydatif choisies dans le groupe constitué par : l'athérothombose, l'athérosclérose, le diabète, les maladies cardiovasculaires, l'inflammation chronique, l'accident vasculaire cérébral et le choc septique, le cancer ; le vieillissement, de préférence de l'athérothrombose.

5. Composition pharmaceutique comprenant une molécule destinée à être utilisée comme défini dans l'une quelconque des revendications 1 à 3 ou composition destinée à être utilisée selon la revendication 4 et au moins un véhicule pharmaceutiquement acceptable, destinée à être utilisée dans le traitement et/ou la prévention de maladies dégénératives associées au stress oxydatif choisies dans le groupe constitué par : l'athérothrombose, l'athérosclérose, le diabète, les maladies cardiovasculaires, l'inflammation chronique, l'accident vasculaire cérébral et le choc septique, le cancer, le vieillissement, de préférence de l'athérothrombose.

6. Cellule hôte génétiquement modifiée transduite, transformée ou transfectée avec et capable d'exprimer dans des conditions appropriées l'ARNsh ou l'ARNsi ou un de leurs dérivés fonctionnels tel que défini dans la revendication 3 destiné à être utilisé dans le traitement et/ou la prévention de maladies dégénératives associées au stress oxydatif, de préférence de l'athérothrombose.
